# EUROPEAN PATENT SPECIFICATION

(11) **EP 4 017 844 B1**
(45) Date of publication and mention of the grant of the patent: **24.01.2024**
(21) Application number: 20760829.0
(22) Date of filing: 20.08.2020
(51) Int. Cl.: C07D 233/64, C07D 263/32, C07D 277/28, C07D 413/04, C07D 417/04, A61P 35/00, A61P 31/00

(54) **COMPOUNDS AND USE THEREOF FOR THE TREATMENT OF INFECTIOUS DISEASES AND CANCER**
VERBINDUNGEN UND VERWENDUNG DAVON ZUR BEHANDLUNG VON INFEKTIONSKRANKHEITEN UND KREBS
COMPOSITIONS ET LEUR UTILISATION POUR LE TRAITEMENT DE MALADIES INFECTIEUSES ET DU CANCER

(30) Priority: 21.08.2019 EP 19192793
(43) Date of publication of application: 29.06.2022
(73) Proprietor: AC BioScience SA, 1066 Epalinges (CH)
(72) Inventor: AUCLAIR, Christian, 78730 Saint-Arnoult en Yvelines (FR); IVES, Annette, 1132 Lully (CH)
(74) Representative: KATZAROV S.A.
(86) International application number: PCT/EP2020/073413
(87) International publication number: WO 2021/032857

(56) References cited:
- EP-A1- 2 537 831
- EP-A1- 2 727 926
- EP-A1- 2 962 698
- WO-A1-2009/144494
- WO-A1-2009/158011
- WO-A1-2010/022217
- WO-A1-2010/051353
- WO-A1-2013/163279
- WO-A1-2019/034768
- US-A1- 2014 051 729
- SINENKO V O ET AL: "Synthesis of new 1,3-thiazole derivatives from 2(5)-hydroxyalkyl-1,3-thiazole-5(2)-carbal dehydes", RUSSIAN JOURNAL OF GENERAL CHEMISTRY, M A I K NAUKA - INTERPERIODICA, RU, vol. 86, no. 7, 17 August 2016 (2016-08-17), pages 1597-1603, XP036032379, ISSN: 1070-3632, DOI: 10.1134/S1070363216070100 [retrieved on 2016-08-17]
- JEFFREY T. BAGDANOFF ET AL: "Inhibition of Sphingosine 1-Phosphate Lyase for the Treatment of Rheumatoid Arthritis: Discovery of ( E )-1-(4-((1 R ,2 S ,3 R )-1,2,3,4-Tetrahydroxybutyl)-1 H -imidazol-2-yl)ethanone Oxime (LX2931) and (1 R ,2 S ,3 R )-1-(2-(Isoxazol-3-yl)-1 H -imidazol-4-yl)butane-1,2,3,4-tetraol (LX2932)", JOURNAL OF MEDICINAL CHEMISTRY, vol. 53, no. 24, 22 November 2010 (2010-11-22), pages 8650-8662, XP055032796, ISSN: 0022-2623, DOI: 10.1021/jm101183p
- SANTUCCI M B ET AL: "Sphingosine 1-phosphate promotes antigen processing and presentation to CD4+ T cells in Mycobacterium tuberculosis-infected monocytes", BIOCHEMICAL AND BIOPHYSICAL RESEARCH COMMUNICATIONS, ELSEVIER, AMSTERDAM, NL, vol. 361, no. 3, 28 September 2007 (2007-09-28), pages 687-693, XP022624909, ISSN: 0006-291X, DOI: 10.1016/J.BBRC.2007.07.087 [retrieved on 2007-08-11]
- GARG SANJAY K ET AL: "SPHINGOSINE 1-PHOSPHATE INDUCES ANTIMICROBIAL ACTIVITY BOTH IN VITRO AND IN VIVO", THE JOURNAL OF INFECTIOUS DISEASES, UNIVERSITY OF CHICAGO PRESS, US, vol. 189, no. 11, 1 June 2004 (2004-06-01) , pages 2129-2138, XP008070182, ISSN: 0022-1899, DOI: 10.1086/386286
- DATABASE REGISTRY [Online] CHEMICAL ABSTRACTS SERVICE, COLUMBUS, OHIO, US; 16 September 2018 (2018-09-16), CHEMCATS: "Various compounds", XP055744852,
- DATABASE REGISTRY [Online] CHEMICAL ABSTRACTS SERVICE, COLUMBUS, OHIO, US; 7 August 2017 (2017-08-07), Aurora Fine chemicals: "Chemical catalog", XP055747549,

## Description

### FIELD OF THE INVENTION

The invention provides an imidazole compound and use thereof in methods for treating a disease or a disorder, such as infectious diseases and cancer, wherein inhibition of sphingosine-1-phosphate lyase is beneficial to treat the disease or the disorder.

### BACKGROUND OF THE INVENTION

Sphingosine-1-phosphate (S1P) is a lipid metabolite that is produced from ceramide and other higher order sphingolipids by sphingosine kinase (SphKl and SphK2). S1P levels are tightly controlled through production via SphKs, recycling back into sphingosine by sphingosine phosphatases (SGPP1 and 2) or lipid phosphate phosphatases (LPP1 and 2) and irreversible degradation through sphingosine-1-phosphate lyase (S1PL; also known as SGPL1 in mammals) into phosphoethanolamine (pEtN) and 2-hexadecanal. Sphingosine-1-phosphate lyase is a vitamin B6-dependent enzyme localized in the membrane of the endoplasmic reticulum. Like many products of sphingomyelin, it exhibits a wide range of biological activities. S1P enhances cell growth and promotes proliferation, differentiation, and survival in different cell systems and can inhibit the normal apoptotic response to a variety of stimuli. Many of the activities of S1P are mediated through five closely related G-protein-coupled receptors family (S1PR) which play a crucial role in sphingolipid signalling. Each of these receptors appears to be tissue specific and to have demonstrated roles in the regulation of cell proliferation and survival in mammalian cells and in various cancer types. S1P increases intracellular calcium levels by both the liberation of calcium from internal stores and through activation of a Gi protein-coupled receptor resulting in the opening of a cell surface calcium channel. Among number of biological effects, S1P signaling is a potent effector of vascular cell adhesion and motility that are important for blood vessel formation. Most data support the concept that S1P signaling via specific S1P receptors, mainly S1PR1, and to a lesser extent S1PR3, orchestrates key events along the continuum of blood vessel formation. Substantial evidence exists for S1P serving as a pro-angiogenic factor as well as an inducer of vascular maturation. A key aspect of the effect of S1P in angiogenesis relates to its ability promote intercellular interactions between endothelial cells and smooth muscle cells that are necessary for the formation of adherent junctions and subsequent stabilization of newly formed blood vessels. In tumor microenvironment this S1P property leads to a vasculature normalization improving the immunological status of the oxygen-enriched tumor and improving responses to conventional anticancer therapies including radiotherapy and chemotherapy. Taking together, it can be concluded that a high concentration of S1P in blood and tissues may have beneficial effects in certain pathological circumstances.

In addition to its role in angiogenesis, S1P and its metabolites 2-hexadecanal and pEtN have clear ability to impact the immune system. Yet, the exact roles are cell type dependent, related to the mechanisms of production and the specific pathobiology of the disease. Many of the effects of S1P are through the modulation of immune cell survival, endocytosis, antigen presentation, redox-dependent stress responses, and the modulation of cell signaling pathways such as Nuclear Factor kappa B (NFkB). Studies have also elucidated roles for 2-hexadecanal and pEtN in stress responses, cell death and cytoskeletal organization.

Mice deficient for S1PL die shortly after birth and have compromised immune systems as demonstrated by their severe inflammatory phenotypes. Similarly mice deficient for both SpHK1 and SpHK2 are embryonically lethal.

It is known that 2-acetyl-4-tetrahydroxybutylimidazole (THI) inhibits S1P lyase activity when administered to mice (Schwab, S. et al., Science 309:1735-1739; 2005). It appeared therefore possible to treat some diseases by inhibiting the enzyme S1P lyase (S1PL).

Pathogenic infections, such as bacterial infections, can be addressed by S1PL modulation of the host and/or the pathogen. Functional SIPLs has been identified in several bacteria, such as *Legionella pneumoniae*, and *Burkholderia spp.*, which cause pulmonary infections of Legionnaire's disease and Melioidosis, respectively.

Modulation of S1P mediated biological actions both at the intracellular and extracellular level can improve pathogen killing within infected immune cells, including the macrophage. For example, in *Leishmania donovani-infected* macrophages, administration of S1P improved parasite clearance while abrogation of SphK activity that produces S1P, and the inhibition of S1PR2 and S1PR2 ligation results in elevated parasite burden within the cells and a modulation of the host immune response.

Additionally, experimental evidence using *leishmania* major deficient for S1PL demonstrate that this molecule can affect the parasite infectivity and delays disease development in mice. This is due to the fact that for this specific parasite they require host-derived sphingolipids for the formation of cell membrane components. Noticeably, also *L. major* parasites deficient for the SphK (required for S1P production) were resistant to infection in a susceptible mouse model. Similarly, *Burkholderia spp.* and *Legionella pneumophilia* have secreted forms of S1PL. *In vivo* infection models using S1PL-deficient bacteria display reduced infection levels and pathogenesis. *In vitro* macrophage studies confirmed that a functional S1PL produced by these bacteria mediate intracellular persistence and modulation of host immune responses to promote survival.

Putative Sphingosine-1-phosphate lyase genes have been found with in many different pathogenic organisms including helminths (e.g.: Schistosoma Masoni), other kinetoplasts (e.g.: Trypanosoma cruzi and Trypanosoma brucei), and species of pathogenic fungi (e.g.: Candida albicans).

Currently leishmaniasis and other neglected tropical diseases (NTDS), which includes, amongst others, Chagas disease and Human African Trypanosomiasis-both caused by infection by *Trypanosoma spp* are of public health concern predominantly in low and low-middle income countries. Annual estimates indicate up to 1 million cases of cutaneous leishmaniasis (CL), 90,000 cases of Visceral leishmaniasis (VL), 7 million cases of Chagas disease. For Human African Trypanosomiasis, approximately 10.5 million people have a med/high risk of infection. These diseases, depending on host genetics and the infecting parasite, can be fatal, if left untreated. Although some forms of CL may be a self-limiting, there is the possibility for the development of the life-threatening secondary disease of mucocutaneous leishmaniasis (MCL) and the development on non-healing progressive CL lesions that require treatment. For example, even though CL may ultimately be resolved it is often with significant amount of scarring.

Thus modulation of sphingosine-1-phosphate (S1P) activity for the treatment of related diseases or disorders, such as infectious diseases and cancer, is still an unmet need. More in particularly, potent and effective S1P lyase inhibitors that modulate the levels S1P and its metabolites 2-hexadecanal and pEtN are needed.

### SUMMARY OF THE INVENTION

An aspect of the present invention provides a compound 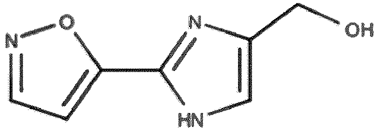 or phosphorylated derivatives thereof and/or phosphoric acid esters thereof.

Another aspect of the present invention provides a pharmaceutical composition comprising the compound of the invention and pharmaceutically acceptable excipients and/or carriers.

Another aspect of the present invention provides the compound of the invention for use in the treatment, prevention or suppression of diseases and disorders known to be susceptible to variations in the levels of sphingosine-1-phosphate, 2-hexadecanal levels and phosphoethanolaminea.

Another aspect of the present invention provides the compound of the invention for use in a method of preventing and/or treating infectious diseases in a subject, wherein the infectious diseases are caused by pathogenic microorganisms selected from the group comprising bacteria, viruses, fungi and parasites.

Another aspect of the present invention provides the compound of the invention for use in a method of treatment of cancer, wherein a therapeutically effective amount of the compound of the invention is administered to a subject prior to or during the chemotherapy and/or the radiotherapy.

### BRIEF DESCRIPTION OF THE FIGURES

The following Figures are not according to the invention and are present for illustration purposes only.

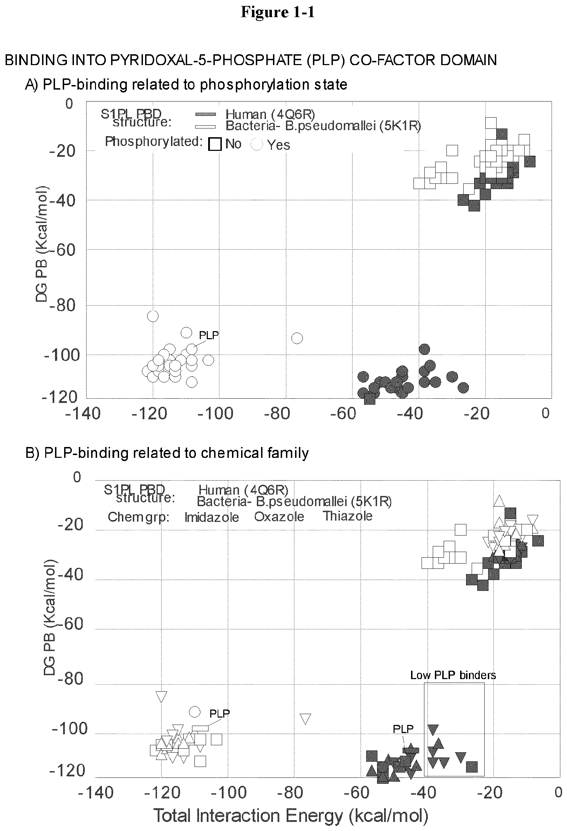

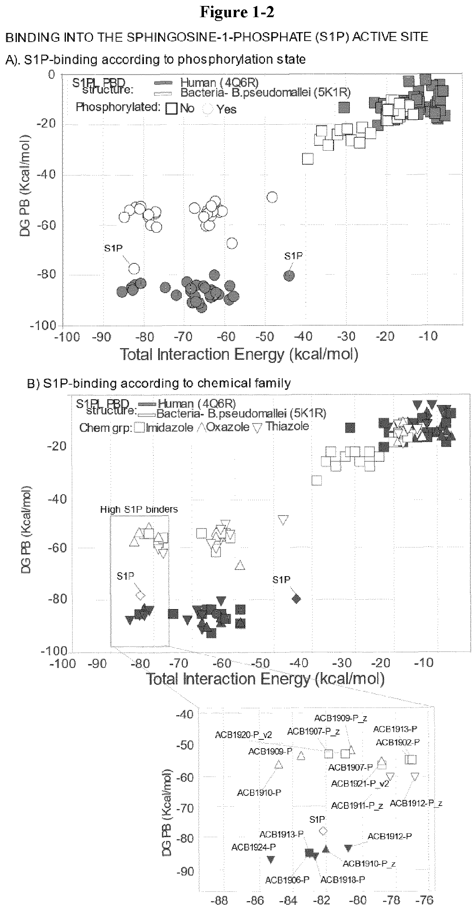

**Figure 1** shows modelling inhibitors into the S1P active site of known S1PL crystal structures. Human S1PL (PDB code: 4Q6R) activated with Pyridoxal-5-phosphate (PLP) and bacterial Burkholderia pseudomallei K96243 sphingosine-1-phosphate lyase with unattached PLP (PDB code: 5K1R). Where relevant the PLP cofactor was removed in Discovery studio. Comparison of the human and bacterial active sites was confirmed to have a high similarity given to an average RMSD 0.5. For the drug both pro-drugs and phosphorylated forms where analyzed and two mechanisms were investigated. Figure 1-1 - Docking of the drugs into the PLP-cofactor domain and Figure 1-2 - Docking of the drugs into the S1P catalytic site. Discovery programme, GOLD and CHEMPLP scoring was used to establish favorable ligand poses; scoring established using chemscore, mm_PBSA and CHARMM. For selection the DeltaG PB (the energy required for ligand binding) was compared to the total interaction energy all residues within the binding site when bound. In all analysis, the S1P and PLP the natural ligand for each site was used for comparisons. Data presented where human S1PL protein in grey and B. pseudomallei in white. Marker shapes represent A) the binding scorings based on phosphorylation sites of each drug; B) the chemical family of imidazole compounds, thiazole compounds, oxazole compounds.

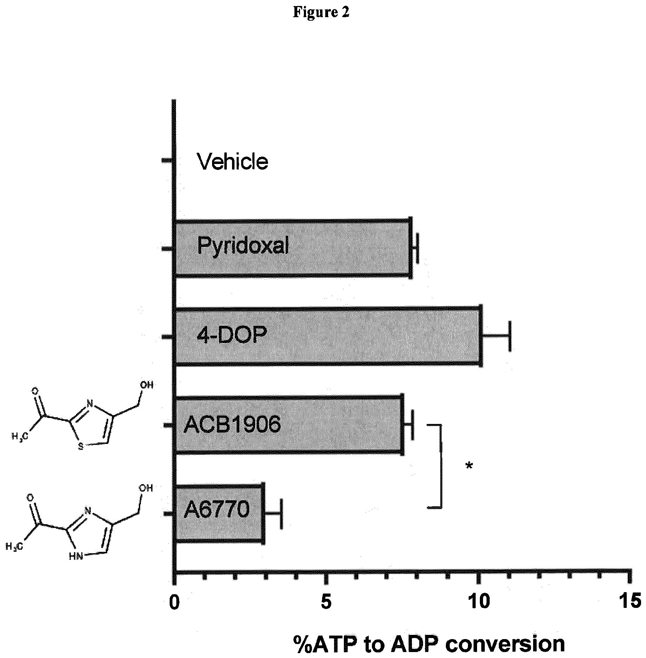

**Figure 2** shows *in vitro* phosphorylation of S1PL inhibitors by human Pyridoxal kinase (PDXK) :
   ACB1906, A6770, 4-DOP (200µM) and pyridoxal HCL (1mM) were incubated with 10µg/ml rhPDXK for 1 hour at 37°C in the presence of 1mM ATP containing- 10mM HEPES buffer with 0.05% Triton X-100, 0.01% Plurogenic F127, 25µM Na₃VO₄ and 250µM MgCl₂. ADP formation was measured using the luminescence-based ADPglo kit from Promega. Statistical significance determined by Anova. * denotes p≤0.05.

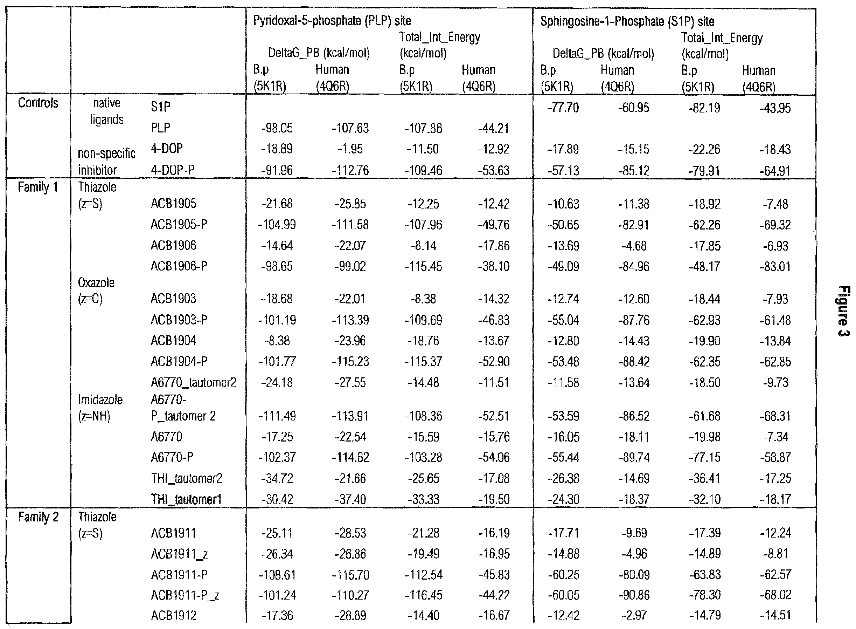

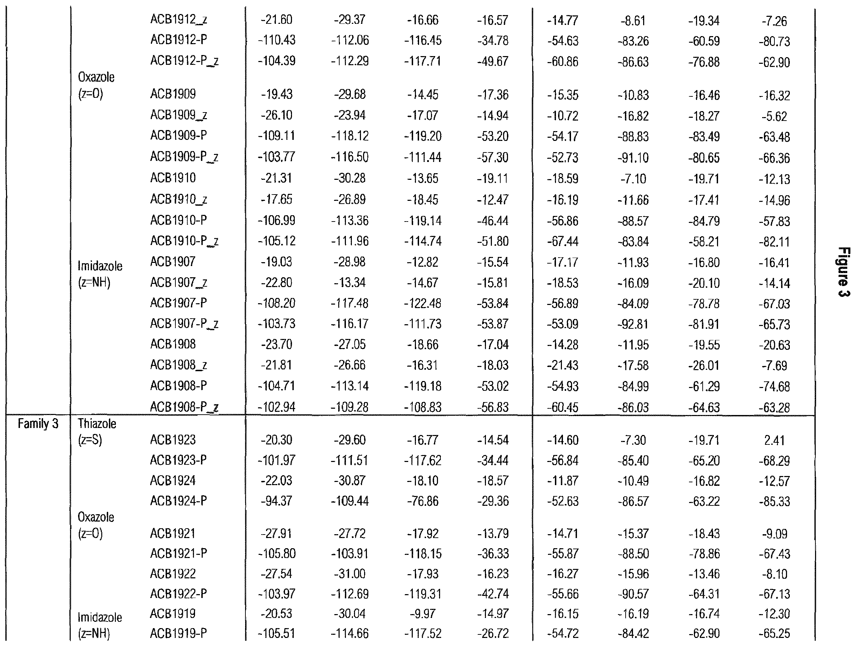

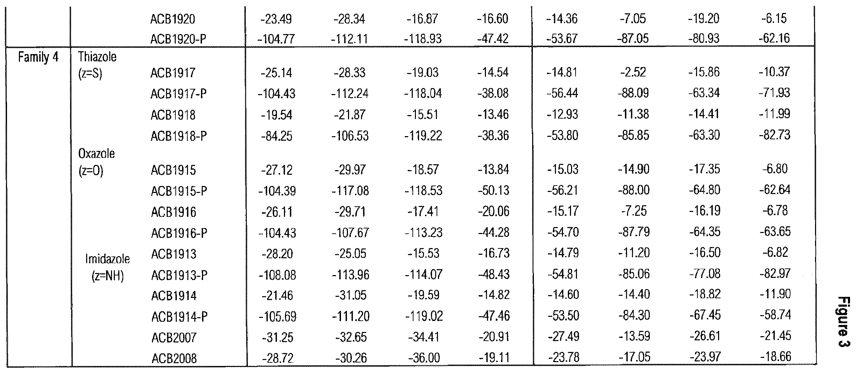

**Figure 3** shows summarized results from molecular modelling. Modelling of the chemical compounds onto human (PBD code: 4Q6R) and bacterial *B. pseudomallei* S1PL (PBD code: SK1R) in either the S1P catalytic domain or within the PLP cofactor domain.

### DETAILED DESCRIPTION OF THE INVENTION

The materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise" is generally used in the sense of include, that is to say permitting the presence of one or more features or components. Also as used in the specification and claims, the language "comprising" can include analogous embodiments described in terms of "consisting of" and/or "consisting essentially of".

As used in the specification and claims, the singular form "a", "an" and "the" include plural references unless the context clearly dictates otherwise.

As used in the specification and claims, the term "and/or" used in a phrase such as "A and/or B" herein is intended to include "A and B", "A or B", "A", and "B".

The compound of the present invention can exist in a tautomeric form which is also intended to be encompassed within the scope of the present invention. "Tautomers" refers to compounds whose structures differ markedly in the arrangement of atoms, but which exist in easy and rapid equilibrium. It is to be understood that the compound of present invention may be depicted as different tautomers. It should also be understood that when the compound has tautomeric forms, all tautomeric forms are intended to be within the scope of the present invention, and the naming of the compounds does not exclude any tautomeric form. Tautomers exist as mixtures of a tautomeric set in solution. In solid form, usually one tautomer predominates. Even though one tautomer may be described, the present invention includes all tautomers of the compound disclosed herein. A tautomer is one of two or more structural isomers that exist in equilibrium and are readily converted from one isomeric form to another. This reaction results in the formal migration of a hydrogen atom accompanied by a shift of adjacent conjugated double bonds. In solutions where tautomerization is possible, a chemical equilibrium of the tautomers can be reached. The exact ratio of the tautomers depends on several factors, including temperature, solvent, and pH. The concept of tautomers that are interconvertible by tautomerizations is called tautomerism. Tautomerizations are catalyzed by: Base: 1. deprotonation; 2. formation of a delocalized anion (e.g., an enolate); 3. protonation at a different position of the anion; Acid: 1. protonation; 2. formation of a delocalized cation; 3. deprotonation at a different position adjacent to the cation.

As used herein the terms "subject" and "patient" are well-recognized in the art, and, are used herein to refer to a mammal, including, for example, humans, animals, domestic pets, livestock and other farm animals; the most preferably a mammal is a human. In some embodiments, the subject is a subject in need of treatment or a subject being infected by pathogens and/or parasites. The term does not denote a particular age or sex. Thus, adult and newborn subjects, whether male or female, are intended to be covered.

As used herein the term "pharmaceutically acceptable excipients and/or carriers" means that the compositions or components thereof so described are suitable for suitable for administration to subject (patient) body without undue toxicity, incompatibility, instability, irritability, allergic response, and the like.

The term "treat" and its grammatical variants (for example "to treat," "treating," and "treatment") refer to administration of an active pharmaceutical ingredient to a subject (patient), such as the compound of the invention, with the purpose of inhibiting a disease or a disorder (such as infectious disease or cancer) in a subject (patient). Treating the disease or the disorder includes ameliorating at least one symptom, reducing severity, impeding progress, and/or curing the subject (patient) of the disease or the disorder. In the present context, treatment entails oral, parenteral, mucosal and/or topical administration of the compounds of the invention or the pharmaceutical composition of the invention to a subject (patient).

As used herein, the term "preventing" refers to prophylactic steps taken to reduce the likelihood of a subject from contracting or suffering from a particular disease or disorder (such as infectious disease or cancer). The likelihood of the disease or the disorder occurring in the subject need not be reduced to zero for the preventing to occur; rather, if the steps reduce the risk of a disease or a disorder across a population, then the steps prevent the disease or the disorder within the scope and meaning herein.

As used herein the term "administration" or "administering" to a subject refers to any means of introducing the compound of the invention or the pharmaceutical composition of the invention onto and/or into the subject (patient) body or a portion thereof. According to an embodiment for administration to animals, the compounds and pharmaceutical composition of the invention may be provided through the food administered to the subject.

The term "therapeutically effective amount," as used herein, refers to any amount of a compound of the invention that will cause inhibition of a disease or a disorder (such as infectious disease or cancer) in a subject and thus reduction of symptoms, disappearance of the symptoms or relief from symptoms related to a disease or a disorder (such as infectious disease or cancer), when applied, either once, or repeatedly over time. Therapeutically effective amounts can be readily determined by persons skilled in the art using routine experimentation and using tests and measures commonly employed in the art, or can be based upon the subjective response of patients undergoing treatment.

The term "prophylactically effective amount," as used herein, is intended to include the amount of a compound of the invention that, when administered to a subject who does not yet experience or display symptoms of a disease or a disorder, such as infectious disease), but who may be predisposed to the disease or the disorder, is sufficient to prevent or ameliorate the disease or the disorder or one or more symptoms of the disease or the disorder. Ameliorating the disease or the disorder includes slowing the course of the disease or the disorder or reducing the severity of later-developing disease or disorder. The "prophylactically effective amount" can be readily determined by persons skilled in the art using routine experimentation and using tests and measures commonly employed in the art, or can be based upon the subjective response of patients undergoing treatment.

The term "neoadjuvant", as used herein, refers to administration of the compound of the invention prior to or during the main chemotherapeutical and/or radiotherapeutical treatment, with the intent of boosting the efficacy or increasing the effectiveness of the chemotherapy and/or radiotherapy.

The present invention provides a compound or tautomers thereof, that can be used as inhibitors of S1P lyase. The present invention also includes pharmaceutically acceptable salts of said compound and tautomers.

An aspect of the present invention provides a compound 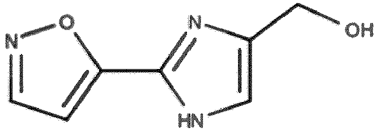 or phosphorylated derivatives thereof and/or phosphoric acid esters thereof.

In some embodiments, the compound of the invention is selected from

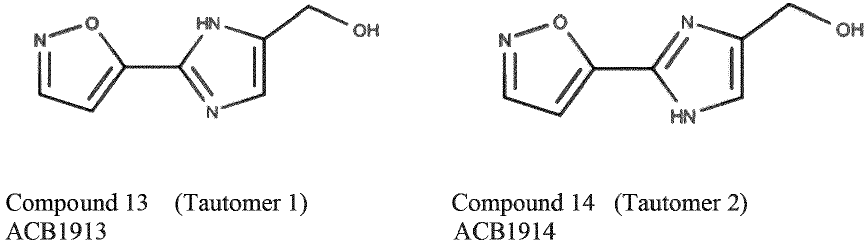

The compound of the invention can be prepared by methods known in the art.

The compound or tautomers thereof, or pharmaceutically acceptable salts of said compound or tautomers disclosed herein can have asymmetric centres. The compound or tautomers thereof, or pharmaceutically acceptable salts of said compound or tautomers of the present invention containing an asymmetrically substituted atom can be isolated in optically active or racemic forms. It is well known in the art how to prepare optically active forms, such as by resolution of racemic forms or by synthesis from optically active starting materials. Cis and trans geometric isomers of the compound or tautomers thereof, or pharmaceutically acceptable salts of said compound or tautomers of the present invention are described and can be isolated as a mixture of isomers or as separate isomeric forms. All chiral, diastereomeric, racemic, and geometric isomeric forms of a structure are intended, unless specific stereochemistry or isomeric form is specifically indicated. All processes used to prepare the compound or tautomers thereof, or pharmaceutically acceptable salts of said compound or tautomers of the present invention and intermediates made herein are considered to be part of the present invention. All tautomers of shown or described compound are also considered to be part of the present invention.

Specifically, the compound of the invention can contain one or more asymmetric centres and can thus occur as racemates and racemic mixtures, single enantiomers, diastereomeric mixtures and individual diastereomers. The present invention is meant to comprehend all such isomeric forms of the compound. The compound of the invention may be separated into their individual diastereoisomers by, for example, fractional crystallization from a suitable solvent, for example methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration. Alternatively, any stereoisomer of a compound of the invention may be obtained by stereospecific synthesis using optically pure starting materials or reagents of known absolute configuration. If desired, racemic mixtures of the compounds may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography. The coupling reaction is often the formation of salts using an enantiomerically pure acid or base.

The diasteromeric derivatives (see Table 1) may then be converted to the pure enantiomers by cleavage of the added chiral residue. The racemic mixture of the compounds can also be separated directly by chromatographic methods utilizing chiral stationary phases, which methods are well known in the art.

**Table 1. Tautomers and regioisomers of the compound of the invention**

| Compound No. | Tautomer (Tau.) | Regioisomer (Reg.) | Ring |
|---|---|---|---|
| 13 -ACB1913 | 1 | | imidazole |
| 14 - ACB1914 | 2 | | imidazole |

The compound or tautomers thereof, or pharmaceutically acceptable salts of said or tautomers disclosed herein have at least one side chain that is typically an aliphatic primary alcohol such as -(CH₂)ₙ-OH, were n is 1 to 5, preferably 1, that can be phosphorylated by PKA and/or by other enzymes, such as pyridoxal kinase, during metabolization of the compound of the invention in the human body and/or in pathogenic microorganisms, thereby providing phosphorylated derivatives of the compound of the invention.

Phosphoric acid esters of the compound of the invention can be hydrolysed by esterases (hydrolase enzymes) during metabolization of the phosphoric acid esters of the compound of the invention in the human body and/or in pathogenic microorganisms in order to provide phosphorylated derivates of the compound of the invention that have biological importance and activity. Phosphoric acid esters of the compound of the invention can be monoesters or diesters. Examples of such phosphoric acid esters, that are not according to the invention and that are present for illustration purposes only, are
Monomethyl ester (1) 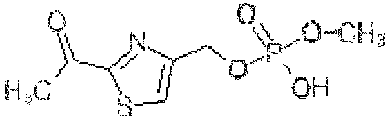
Monomethyl ester (2) 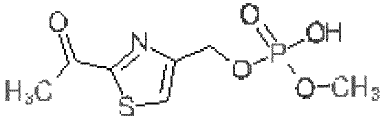
Dimethyl ester

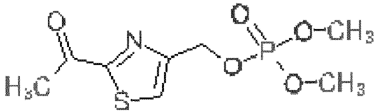

This is the first time that the compound of the invention have been shown to bind to bacterial S1PL.

Another aspect of the present invention provides a pharmaceutical composition comprising the compound of the invention and pharmaceutically acceptable excipients and/or carriers.

Certain pharmaceutical compositions of the invention are single unit dosage forms suitable for oral or mucosal (such as nasal, sublingual, vaginal, buccal, or rectal) administration to a patient. Examples of dosage forms include, but are not limited to tablets, caplets, capsules, such as soft elastic gelatine capsules, cachets, troches, lozenges, dispersions, suppositories, powders, solutions, aerosols (such as nasal sprays or inhalers), liquid dosage forms suitable for oral or mucosal administration to a patient, including suspensions (such as aqueous or non-aqueous liquid suspensions, oil-in-water emulsions, or a water-in-oil liquid emulsions), solutions, and elixirs.

The formulation of the pharmaceutical composition of the invention should suit the mode of administration. For example, oral administration requires enteric coatings to protect the compound of this invention from degradation within the gastrointestinal tract. Similarly, a formulation may contain ingredients that facilitate delivery of the active ingredient(s) to the site of action. For example, the compound may be administered in liposomal formulations, in order to protect them from degradative enzymes, facilitate transport in circulatory system, and effect delivery across cell membranes to intracellular sites.

The pharmaceutical compositions of the invention suitable for oral administration can be presented as discrete dosage forms, such as, but are not limited to, tablets (such as chewable tablets), caplets, capsules, and liquids (such as flavoured syrups). Such dosage forms contain predetermined amounts of active ingredients, and may be prepared by methods of pharmacy well known to those skilled in the art. See, e.g., Remington's Pharmaceutical Sciences, 18th ed., Mack Publishing, Easton Pa. (1990).

Typical oral dosage forms are prepared by combining the compound of the invention in an intimate admixture with at least one excipient according to conventional pharmaceutical compounding techniques. Excipients can take a wide variety of forms depending on the form of preparation desired for administration.

Because of their ease of administration, tablets and capsules represent the most advantageous oral dosage unit forms. If desired, tablets can be coated by standard aqueous or nonaqueous techniques. Such dosage forms can be prepared by conventional methods of pharmacy. In general, pharmaceutical compositions and dosage forms are prepared by uniformly and intimately admixing the compound of the invention with liquid carriers, finely divided solid carriers, or both, and then shaping the product into the desired presentation if necessary. Disintegrants may be incorporated in solid dosage forms to facility rapid dissolution. Lubricants may also be incorporated to facilitate the manufacture of dosage forms (such as tablets). For example pharmaceutically acceptable excipients particularly suitable for use in conjunction with tablets include, for example, inert diluents, such as celluloses, calcium or sodium carbonate, lactose, calcium or sodium phosphate; disintegrating agents, such as cross-linked povidone, maize starch, or alginic acid; binding agents, such as povidone, starch, gelatin or acacia; and lubricating agents, such as magnesium stearate, stearic acid or talc.

Other pharmaceutical compositions of the invention are transdermal and topical dosage forms administered to a patient. Such dosage forms are selected from the group comprising ophthalmic solutions, sprays, aerosols, creams, lotions, ointments, gels, solutions, emulsions, suspensions, or other forms known to a person skilled in the art (see for example Remington's Pharmaceutical Sciences, 16th and 18th eds., Mack Publishing, Easton Pa. (1980 & 1990); and Introduction to Pharmaceutical Dosage Forms, 4th ed., Lea & Febiger, Philadelphia (1985). Transdermal dosage forms include "reservoir type" or "matrix type" patches, which can be applied to the skin and worn for a specific period of time to permit the penetration of a desired amount of the compounds of the invention.

Suitable excipients (e.g., carriers and diluents) and other materials that can be used to provide transdermal, topical, and mucosal dosage forms are well known to a person skilled in the art, and depend on the particular tissue to which a given pharmaceutical composition or dosage form will be applied.

Depending on the specific tissue to be treated, additional components may be used prior to, in conjunction with, or subsequent to treatment with the compound of the invention. For example, penetration enhancers may be used to assist in delivering the compound of the invention to the tissue.

The pH of a pharmaceutical composition or dosage form, or of the tissue to which the pharmaceutical composition or dosage form is applied, may also be adjusted to improve delivery of the compound of the invention. Similarly, the polarity of a solvent carrier, its ionic strength, or tonicity can be adjusted to improve delivery. Compounds such as stearates may also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery-enhancing or penetration-enhancing agent. Different salts, hydrates or solvates of the active ingredients can be used to further adjust the properties of the resulting composition.

Other pharmaceutical compositions of the invention are parenteral dosage forms administered to patients by various routes including, but not limited to, subcutaneous, intravenous (including bolus injection), intramuscular, and intraarterial. Because their administration typically bypasses patients' natural defenses against contaminants, parenteral dosage forms are specifically sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms of the invention are well known to a person skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

The composition, shape, and type of a dosage form of the pharmaceutical composition of the invention will vary depending on its use. For example, a dosage form used in the acute treatment of a disease, such as infectious diseases or cancer, may contain larger amounts of the compound of the invention than a dosage form used in the chronic treatment of the same disease.

The following aspect, which is not according to the invention and which is present for illustration purposes only provides a method of inhibiting sphingosine-1-phosphate lyase activity in a subject and/or in pathogenic microorganisms selected from the group comprising bacteria, viruses, fungi and parasites, preferably bacteria, fungi and parasites, the method comprising using the compound of the invention. A specific embodiment, that is not according to the invention and that is present for illustration purposes only, provides a method of inhibiting sphingosine-1-phosphate lyase activity in a subject, the method comprising using the compound of the invention. Using the compound of the invention includes administering the compound of the invention to the subject. Another specific embodiment, that is not according to the invention and that is present for illustration purposes only, provides a method of inhibiting sphingosine-1-phosphate lyase activity in pathogenic microorganisms selected from the group comprising bacteria, viruses, fungi and parasites, preferably bacteria, fungi and parasites, the method comprising using the compound of the invention. Using the compound of the invention includes contacting the pathogenic microorganisms with the compound of the invention. In another specific embodiment, that is not according to the invention and that is present for illustration purposes only, the subject can be infected with the pathogenic microorganism and provides a method of inhibiting sphingosine-1-phosphate lyase activity in a subject and in pathogenic microorganisms selected from the group comprising bacteria, viruses, fungi and parasites, preferably bacteria, fungi and parasites, the method comprising using the compound of the invention. Using the compound of the invention includes administering the compound of the invention to the subject infected with the pathogenic microorganisms.

A specific embodiment, that is not according to the invention and that is present for illustration purposes only, provides a use of the compound of the invention for inhibiting S1P lyase in a subject and/or in pathogenic microorganisms selected from the group comprising bacteria, fungi and parasites. In preferred embodiments, the thiazole and/or oxazole compounds of the invention are used.

The compound of the invention is inhibitor of S1P lyase and thus also S1P level enhancing agent and interferes with the action of S1PL in mammalian cells. Aside from elevating S1P level, inhibition of S1P lyase has also influence on other biological effects, such as the products of S1P lyase: 2-hexadecanal and phosphoethanolamine (pEtN) which can influence cellular responses and survival. Thus the compound of the invention is useful in the treatment, prevention or suppression of diseases and disorders known to be susceptible to variations in the levels of sphingosine-1-phosphate, 2-hexadecanal levels and phosphoethanolamine (pEtN), such as infectious diseases and cancer.

The compound of the invention increases the amount of S1P, while decreasing the levels of its metabolites. Thus the compound of the invention would also make use of the immunomodulatory capabilities of S1P, and its metabolites, and boost the development of protective immune responses in the infected host (a subject affected by the infectious diseases). In preferred embodiments, the thiazole and/or oxazole compounds of the invention are used.

Another aspect of the present invention provides the compound of the invention for use in the treatment, prevention or suppression of diseases and disorders known to be susceptible to variations in the levels of sphingosine-1-phosphate, 2-hexadecanal levels and phosphoethanolaminea.

Without being limited by theory and in addition to acting on host cells, including those of the immune system, the compound of the invention can target and affect pathogen-derived S1PL, which is a virulence factor for pathogenic infections, and thus affect survival of pathogenic microorganisms. The compound of the invention can therefore inactivate or inhibit essential survival processes of pathogenic microorganisms, which means that the compound of the invention have the ability to decrease, suppress, attenuate, diminish, or arrest the development or progression of a pathogen-mediated infectious diseases in a subject.

Indeed, besides the presence of S1PL in humans, S1PL is also present in parasites, such as *Leishmania spp.* or *Schistosoma spp.*, in bacteria, such as *Burkeholderia pseudomallei* or *Legionella pneumophilia*, as well as in pathogenic fungi and therefore may be targeted (inhibited) by the compound of the present invention.

Infectious diseases are caused by pathogenic microorganisms selected from the group comprising bacteria, viruses, fungi and parasites. They are contagious and transmitted by insects, animals, person-to-person and/or by consuming contaminated food and/or water.

The treatment strategy therefore includes treatment with the compound of the invention (as S1PL inhibitors) through the oral, topical and parenteral route, ideally for a short course after the onset of disease symptoms. Patient populations with infections where low S1P levels are found during infection and whose elevation confers protection and a reduction in clinical pathology, can be typically treated with the compound of the invention.

Thus another aspect of the present invention provides a compound of the invention for use in a method for treating and/or preventing infectious diseases in a subject, wherein the infectious diseases are caused by pathogenic microorganisms selected from the group comprising bacteria, viruses, fungi and parasites (including protozoa and helminths). In another specific embodiment, the present invention provides a compound of the invention for use in a method of treating infectious diseases in a subject, wherein the infectious diseases are caused by pathogenic microorganisms selected from the group comprising bacteria, viruses, fungi and parasites (including protozoa and helminths). In some embodiments, the pathogen-derived S1PL is a virulence factor. Specifically, in the case of Leishmaniasis and Chagas disease and African Trypanosomiaisis (caused by *Trypanosoma spp.*) they require sphingolipid biosynthetic pathways for the generation of their cell membranes and to produce virulence-associated lipid structures. Phosphoethanolamine (pEtN) the product of S1P catabolism is required for phosphatidylethanolamine and phoshatidylcholine membrane components.

According to some embodiment of the invention, the infectious diseases are selected from the group comprising pulmonary infections (selected from the group comprising virus-induced infections, such as Influenza, Respiratory syncytial virus, SARS-CoV, Bacteria-induced pneumonia and Tuberculosis), plague, systemic infections (selected from the group comprising Sepsis and those induced by Cytomegalovirus, Meliodosis, Legionnaire's disease, Dengue, Chikagunya, Measles, Candidiasis) and parasitic diseases (selected from the group comprising Leishmaniasis, Human African Trypanosomiasis, Chagas disease Schistosomiasis, Echinococcosis, and Malaria).

The amount of the compound of the invention, dosing schedule, and route of administration will vary depending on the drug, the patient and the pathogenic microorganism, and can readily be determined by those of ordinary skill in the art.

In the context of the present invention, the subject may be selected from the group consisting of a mammal, a fish, a bird, and a crustacean. The mammal may be selected from the group consisting of a human, a bovine, an equine, a canine and an ungulate. The fish may be selected from the group consisting of a hagfish, a lamprey, a cartilaginous fish and a bony fish. The bird may be selected from the group consisting of chicken, a turkey, and a fowl. The crustacean may be selected from the group consisting of a shrimp, a crab, a lobster, a langouste.

In the context of the present invention, pathogenic microorganisms selected from the group comprising bacteria, viruses, fungi and parasites (including helminths and protozoa). The pathogenic microorganisms can have functional S1PL or not.

According to some preferred embodiments of the invention, pathogenic bacteria are selected from the group comprised of the following genuses of *Mycobacterium*, *Listeria*, *Legionella.*, *Burkholderia, Escherichia, Brucella, Bordatella, Helicobacter, Yersinia, Streptococcus*, *Staphylococcus, Clostridium, Pseudomonas, Diptheria*, *Klebsiella*, *Campylobacter*, *Acetinobacter, Cryptococcus, Clostridioides, Enterococcus, Shigella, Neisseria, Salmonella, Treponema, Cryptosporidium and Wolbachia*

According to some preferred embodiments of the invention, pathogenic fungi are selected from the group comprised of the following genuses of *Candida*, *Aspergillus*, *Fusarium*, *Cryptococcus*

According to some preferred embodiments of the invention, parasites including helminths are selected from the group comprising of the following genuses of *Leishmania*, *Trypanosoma*, *Schistosoma, Taenia, Echinococcus, Toxoplasma, Wuchereria, Brugia*, *Fasciola, Onchocerca*, *Dracunculus*, and *Plasmodium.*

According to some preferred embodiments of the invention, viruses are selected from the group comprising
- single-stranded positive sense RNA viruses, such as Flaviridae, Hepeviridae, Picornaviridae, Togaviridae
- single-stranded negative sense RNA viruses, such as Filoviridae, Paramyxoviridae, Rhabdoviridae
- double stranded RNA viruses, such as Reoviridae.
- double stranded DNA viruses, such as Herpesviridae, and Poxviridae.

The compound of the invention are useful in treating infectious diseases, for example preventing, inhibiting and/or ameliorating symptom of an infectious disease.

According to some embodiments of the invention, one or more active agents in addition to a compound of the invention can be used in methods of preventing and/or treating infectious diseases. The one or more additional active agents selected can be selected from the group comprising antibiotics, anti-bacterial agents, antifungal agents, antiparasitic agents, osmotic diuretics, anti-convulsants, anti-pyretics, immunomodulating drugs (including: anti-oxidants, anti- inflammatory drugs, immunosuppressants and immunostimulatory drugs), anti-viral agents and combinations thereof.

Examples of such additional active agents include:
- antibiotics and anti-bacterial agents, which include chemical agents that are active against bacteria. In common usage, an antibiotic is a substance or compound that kills or inhibits the growth of bacteria. Anti-bacterial antibiotics can be categorized based on their target specificity: "narrow-spectrum" antibiotics target particular types of bacteria, such as Gram-negative or Gram-positive bacteria, while broad-spectrum antibiotics affect a wide range of bacteria. Antibiotics which target the bacterial cell wall (such as penicillins, cephalosporins, cephems), or cell membrane (such as polymixins), or interfere with essential bacterial enzymes (such as quinolones, sulfonamides) usually are bactericidal in nature. Those which target protein synthesis such as the aminoglycosides, macrolides and tetracyclines are usually bacteriostatic. Three newer classes of antibiotics include: cyclic lipopeptides (such as daptomycin), glycylcyclines (such as tigecycline), and oxazolidinones (such as linezolid). Tigecycline is a broad-spectrum antibiotic, while the two others are useful for Gram-positive infections.
- antifungal agents, which include therapeutic compounds or bioactive agents that may be used to treat a fungal infection in a subject. An antifungal drug is a medication used to inhibit the growth of, or destroy fungi. Antifungal agents include, for example, polyene antifungals, imidazole, triazole and thiazole antifungals, allylamines, echinocandins, griseofulvin, flycystosine, undecylenic acid, among others.
- antiparasitic agents, which include therapeutic compounds or bioactive agents that are used to treat parasitic diseases including nematodes, cestodes, trematodes, infectious protozoa, and amoebas. Exemplary antiparasitic agents include: anti-nematodes (such as mebendazole, pyrantel pamoate, thiabendazole, diethycarbazine), anti-cestodes (such as niclosamide, praziquantel), anti-trematodes (such as praziquantel), anti-amoebics (such as rifampin and amphotericin B), anti-protozoals (such as melarsoprol, eflomithine, metronidazole, miltephosine, glucantime, paromycin sulfate,, Artimisinin, pentavalent antimonials, pentamidine, nifurtimox, Amphotericin B and tinidazole (and other nitroimidazole-based drugs), among others.
- anti-viral agents that includes virus adsorption inhibitors (e.g.: polysulphates, polysulphonates, polycarboxylates, polyoxometalates, zintevir, cosalane derivitives, Tromantine, ), Entry and fusion inhibitors (e.g.: enfuvir, docosanol, enfuvirtide, Maraviroc, Pleconaril, Vicriviroc), Uncoating inhibitors (e.g.: Pleconaril, Amantadine, rimantadine), Viral synthesis inhibitors (including those that affect transcription, reverse transcription, integration of viral genome into host cell genomes, protein synthesis and processing (e.g.: Zidovudin, Lamivudine, Zalcitabine, Nevirapine, Efavirenz, Delavirdines, Saquinavir, Ininavir, fomivirsen, adefovir, atazanavir, mozenavir, tipranavir, abacavir, Acyclovir, Amprenavir,Cidovir, Darunavir, Didanosine Edoxudine,efavirenz,Emtricitabine, Entevavir, Famciclovir, Fosamprenavir, Methisazone,Nelfinavir, Norvir, Nevirapine, Penciclovir, Podophyllotoxin, Raltegravir, Ribavirin, Rimantadine, Ritonavir, Saquinavir, Sofobuvir, Stavudine, Trifluridine,Valaciclovir, ganciclovir, valganciclovir, Vidarabine, Ribavirin, Zalcitabine, azidothymidine)), Viral assembly (e.g.: rifampicin, atazanavir, Fosfonet, Indinavir, Lopinavir, Loviride, tipranavir), Viral release (e.g.: zanamivir, oseltamivir, RWJ270201, Mycophenolic acid, EICAR, VX487), among others.
- osmotic diuretics (such as mannitol and urea),
- anti-convulsants (such as diazepam, phenytoin, phenobarbital, and phenobarbitone),
- anti-pyretics (such as paracetamol),
- Immuno-modulating drugs including anti-oxidants, anti- inflammatory drugs (such as NSAIDS, steroids, cyclosporin, thalidomide, revlimid, anti-TNF antibodies (e.g., infliximab, etanercept), and pentoxifylline), immuno-stimulants (such as Toll-like receptor ligands (e.g.: Imiquimod), immune Receptor agonists, recombinant chemokines and/or cytokines (e.g.: recombinant TNF).

Thus in some embodiments of the invention, a compound of the invention is administered adjunctively with one or more additional active agents. Administration of a compound of the invention and one or more additional active agents may be concurrent (in the same dosage form or in separate dosage forms administered to the patient at approximately the same time), or not.

As described above, some embodiments include co-administering a compound of the invention and one or more additional active agent. By "co-administration" or "co-administering", it is meant that a compound of the invention and the one or more additional active agent are administered in such a manner that administration of a compound of the invention has an effect on the efficacy of the treatment of the one or more additional active agent, regardless of when or how they are actually administered. Thus in one embodiment, a compound of the invention and the one or more additional active agent are administered simultaneously. In one such embodiment, administration in combination is accomplished by combining a compound of the invention and the one or more additional active agent in a single dosage form. In another embodiment, a compound of the invention and the one or more additional active agent are administered sequentially. In one embodiment a compound of the invention and the one or more additional active agent are administered through the same route, such as orally. In another embodiment, a compound of the invention and the one or more additional active agent are administered through different routes, such as one being administered orally and another being administered parenterally and/or topically. In some embodiments, the time period between administration of a compound of the invention and administration of the co-administered one or more additional active agent can be about 1 hour, 2 hours, 3 hours, 5 hours, 8 hours, 10 hours, 12 hours, 15 hours, 1 8 hours, 20 hours, 24 hours, 36 hours, 48 hours, 3 days, 4 days, 5 days, 6 days, 7 days, 10 days, 14 days, 21 days, 28 days, or 30 days.

Contrary to the prior art evidence that S1P pathway inhibition leads to favorable regulation of key cellular processes that contribute to tumor initiation and progression to cancer, the Applicant observed that gemcitabine-treated patients with elevated levels of plasmatic S1P concentrations, showed better overall survival rates. The activation of the S1P receptor 1 by S1P enhances the formation of adherent junctions, inhibits vascular endothelial growth factor signaling, suppresses sprouting and stabilizes new vascular connections.

The method according to the invention pertains to administrating a sphingosine-1-phosphate lyase inhibitor of the present invention as adjuvant or neoadjuvant therapy, i.e. during or prior to chemotherapy and/or radiotherapy. The neoadjuvant therapy according to the invention suppresses the sprouting angiogenesis and stabilizes the blood vessels. Consequently, the following chemotherapeutic agent has a better access to the tumor cells and therefore, an enhanced efficacy in its therapeutic effect. Likewise, the stabilization of the neovascular network improves the efficiency of a sequential anticancer radiotherapy.

A specific embodiment, that is not according to the invention and that is present for illustration purposes only, provides the compound of the invention for use in a method for improving the efficacy of a chemotherapy and/or a radiotherapy, wherein a therapeutically effective amount of the compound of the invention is administered to a subject prior to the chemotherapy and/or the radiotherapy.

Another aspect of the present invention provides the compound of the invention for use in a method of treatment of cancer, wherein a therapeutically effective amount of the compound of the invention is administered to a subject prior to or during the chemotherapy and/or radiotherapy.

In the context of the present invention, the chemotherapy can be the cytotoxic or the cytostatic chemotherapy that is selected from the group consisting of treatment with alkylating agents, topoisomerase inhibitors type 1 and type 2, antimetabolites, tubulin inhibitors, platinoids, and taxanes.

In the context of the present invention, the cancer is a solid tumor cancer displaying an abnormal tumor vasculature including but not limited to pancreatic adenocarcinoma, lung cancer, breast cancer, prostate cancer, cervix squamous cells carcinoma.

The sequential chemotherapy according to the invention involves a single anti-cancer agent or a combination of anti-cancer agents. Preferably, none of these agents displays anti-angiogenic properties. In one embodiment, the sequential chemotherapy involves agents selected from a group consisting of gemcitabine, paclitaxel, cisplatin and oxaliplatin.

In one embodiment, the subject displays a sphingosine-1-phosphate plasmatic concentration less than the median sphingosine-1-phosphate plasmatic concentration value of healthy volunteers. Since the sphingosine-1-phosphate plasmatic concentration measurement is analytical-method-dependent, the plasmatic concentration is measured with the same experimental protocol in the subject and in the healthy volunteers. In one embodiment, the subject displays a sphingosine-1-phosphate plasmatic concentration inferior to 700 nM, preferably inferior to 650 nM.

The foregoing description will be more fully understood with reference to the following Examples. Such Examples, are, however, exemplary of methods of practising the present invention and are not intended to limit the application and the scope of the invention.

### EXAMPLES

### Example 1

The following example is not according to the invention and is present for illustration purposes only.

Examples of synthesis of the following compounds is provided:

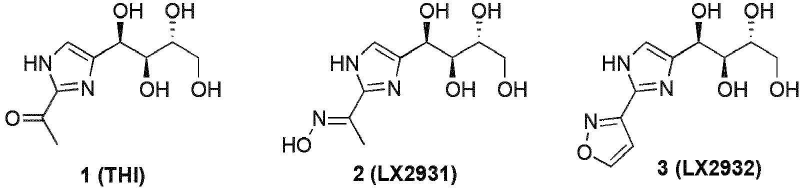

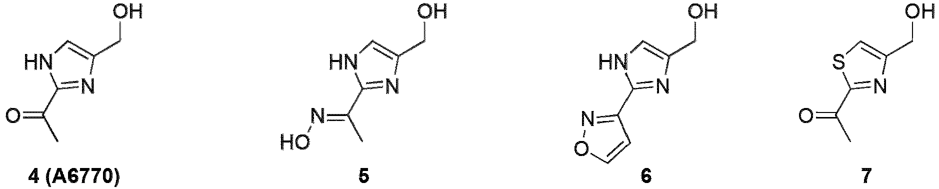

Tetrahydroxylated imidazole **1** (also referred to as THI) was synthesized by condensation of an *α*-aminocarbonyl fragment, fructosamine (shown in its open form), and the imidate formed by the action of sodium methoxide on 2-ethoxyacrylonitrile. 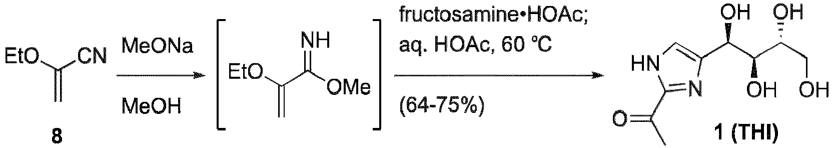 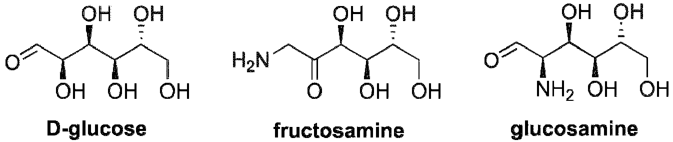 Synthesis of THI (**1**) from 2-ethoxyacrylonitrile and relevant sources of chirality.

With minor deviation from the above procedure, it was found that glucosamine-HCl could serve as a suitable and *commercially available* replacement for fructosamine-HOAc (prepared from D-glucose: 1. Bn₂NH, HOAc, EtOH, reflux; 2. H₂, Pd/C, HOAc/EtOH), albeit with ~15% lower yield (30 mmmol scale, unoptimized). Imidazole **2** (also known in the literature as LX2931) was produced in a straightforward manner from THI by condensation with hydroxylamine. Imidazole **3** (also referred to as LX2932) was obtained analogously to THI by using isoxazole-3-carbonitrile (**9**) as the imidate source. In similar fashion, substitution of fructosamine-HOAc with glucosamine-HCl was successful and resulted in ~20% lower yield (5 mmol scale, unoptimized). 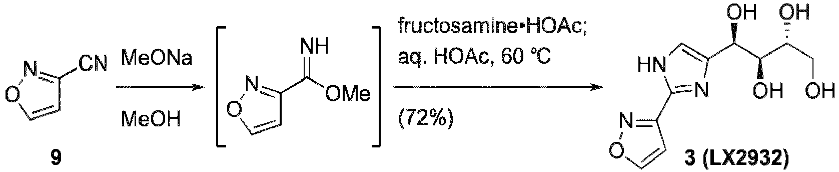 Preparation of LX2932 (3) from isoxazole-3-carbonitrile.

Isoxazole-3-carbonitrile has been previously prepared, but methodology developed by one of the inventors was leveraged with the intent of improving ease and efficiency in its construction. Briefly, ethyl glyoxylate was condensed with hydroxylamine and the resulting oxime oxidized with di(acetoxy) iodo-benzene in a mixture of methanol and ethyl vinyl ether. As it was produced from the oxidation, nitrile oxide **10** underwent [3+2] cycloaddition with the vinyl ether to afford the dihydro-isoxazole (**11**). In a one pot reaction, dihydro-isoxazole **11** was converted to the aryl carboxamide (**12**), which was subsequently dehydrated under standard conditions with phosphorous oxychloride and pyridine, providing pure isoxazole-3-carbonitrile upon vacuum distillation. 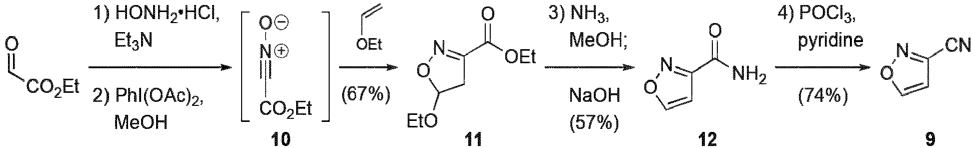 Synthesis of isoxazole-3-carbonitrile using a [3+2]-cycloaddition.

Some experimentation at the amidation-aromatization stage identified conditions that may more effectively convert the dihydro-isoxazole to the aryl carboxamide: 1) 0.5 equiv. DBU, 1.5 M in MeOH, rt, 20 min; 2) 3.5 equivalents of NH₃ (7 N in MeOH), 40 °C, 16 h; work up and silica gel chromatography. The DBU-NH₃ method provided ~10% improvement in yield.

Ideally, imidazole **4** (also known as A6770) would have been accessed directly from THI or its intermediate vinyl ether through oxidative cleavage of the glycol chain with sodium periodate, but isolation of the vinyl ether proved difficult and oxidative cleavage of THI only provided unacceptably low and variable yields of the ketoaldehyde (which would then require differentiation of the two carbonyls). Thus, A6770 was constructed based on previously reported work that started from an already intact imidazole. Commercially available hydroxymethylimidazole **1** was sequentially protected with chlorotriethylsilane and chloromethyl ethyl ether to furnish imidazole **13** as an inconsequential mixture of regioisomers. 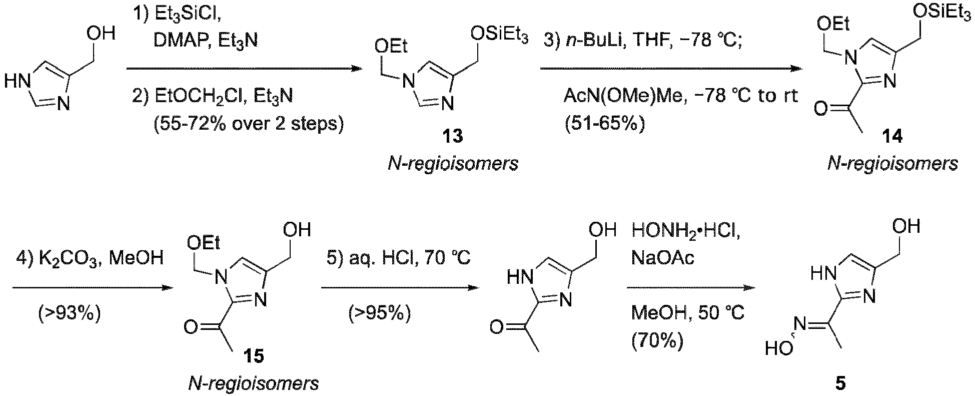 Synthesis of A6670 and imidazole 5 from 4-hydroxymethylimidazole.

This regioisomeric mixture then underwent C2-lithiation and acetylation at -78 °C to afford methyl ketone **14** (also as a mixture of regioisomers) in acceptable yields. Removal of the *O-*silyl group could be carried out with Bu₄NF in THF in 83% yield but was better accomplished with K₂CO₃ in MeOH. The final deprotection step in the synthesis of A6670 (**4**) was achieved by heating hydroxymethylimidazole **15** in aqueous HCl. Analogously to the conversion of THI (**1**) to LX2931 (**2**), A6670 could be transformed to oxime **5** by condensation with hydroxylamine. The added inertness and lipophilicity of the isoxazole of LX2932 (**3**) relative to the acetyl group of THI (**1**) allowed for the synthesis of hydroxymethylimidazole **6** in a 2-step sequenence with NaIO₄ and NaBH₄. 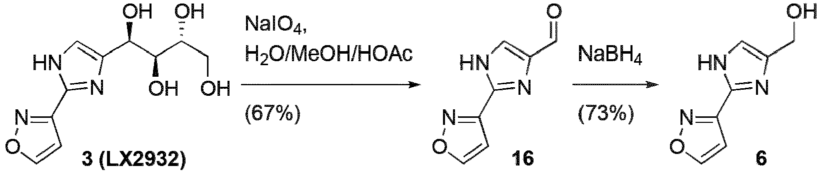 Preparation of imidazole **6** from LX2932.

Lastly, the targeted thiazole (7) was obtained in 4 steps from known thioamide 17. *S*-Alkylation with bromopyruvate, followed by treatment with triflic anhydride and base furnished thiazole **18** in high yield. In a straightforward manner, reduction of the ester and release of the ketone with aqueous hydrochloric acid provided hydroxymethylthiazole 7. 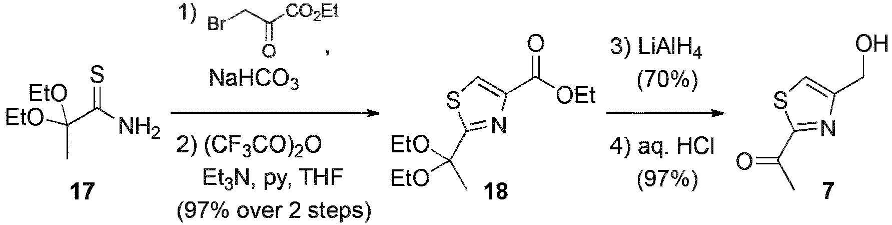 Synthesis of hydroxymethylthiazole 7.

### Experimental Procedures and Data

*General:* Unless otherwise stated, solvents and reagents were purchased from common commercial suppliers and used without further purification. Certain solvents/reagents were treated as follows: water (distilled), THF (distilled from Na/Ph₂CO), MeOH (distilled from I₂-activated Mg turnings), CH₂Cl₂ (distilled from CaH₂), MeCN, (distilled from CaH₂), pyridine (distilled from CaH₂), Et₂NH (distilled from CaH₂), POCl₃ (distilled), trifluoracetic anhydride (distilled), *N*-methoxy-*N*-methylacetamide (fractionally distilled from CaH₂). Unless otherwise stated, reactions were carried out in oven-dried (130 °C) glassware under an atmosphere of argon. Abbreviations used to describe signal shape and multiplicity in NMR are as follows: singlet, "s"; doublet, "d"; triplet, "t"; quartet, "q"; quintet, "quint"; doublet of doublets, "dd"; broad, "br"; apparent, "app". Comparisons to reported literature were made where possible and are in accord with said data in all cases. Nuclear magnetic resonance (NMR) spectra were obtained in deuterated solvents: D₂O (containing 2 drops of aq. 1 M HCl), acetone-*d₆*, DMSO-*d₆*, CD₃OD or CDCl₃. In acquiring NMR spectra in such a manner, it should be noted that there are also signals from the residual solvent as well as adventitious water that also appear: for D₂O, these are 4.80 ppm in ¹H NMR and none in ¹³C NMR; for acetone-*d₆*, these are 2.05 ppm for ¹H NMR and 118.69, 1.39 ppm in ¹³C NMR. 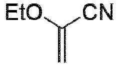 2-Ethoxyacrylonitrile (**8**) - JZ-23-103, 073

Neat trimethylsilylcyanide (24.5 mL, 193 mmol, 1.00 equiv.) was added over ~25 min from an addition funnel to an ice-cold mixture of bromoacetaldehyde diethylacetal (29.9 mL, 193 mmol, 1.00 equiv.) and tin(II) chloride (183 mg, 0.96 mmol, 0.5 mol%) in a round bottom flask. After the addition was complete, the ice bath was removed and the resulting yellow-brown mixture stirred until the starting material was determined to be consumed by ¹H NMR analysis of an aliquot (~3 h), at which point it was diluted with t-BuOMe (100 mL), placed in a room temperature water bath and diethylamine (29.8 mL, 289 mmol, 1.50 equiv.) was added from the same addition funnel over ~15 min. The beige slurry was stirred for a further 2.5 h (consumption of intermediate verified by ¹H NMR) before filtration through Celite^{®} and washing with additional *t*-BuOMe. The filtrate was concentrated on a rotary evaporator under reduced pressure to afford a brown liquid. Fractional distillation under reduced pressure afforded a clear, colourless oil as 2-ethoxyacrylonitrile (12.7 g, 68% yield).

¹H NMR (300 MHz, CDCl₃): δ 4.98 (d, *J* = 3.0 Hz, 1H), 4.88 (d, *J* = 3.0 Hz, 1H), 3.86 (q, *J* = 6.0 Hz, 1H), 1.37 (t, *J* = 7.0 Hz 3H).

¹³C NMR (75.5 MHz, CDCl₃): δ 136.2, 114.8, 100.7, 65.1, 14.0. 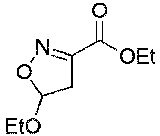 Ethyl 5-ethoxy-4,5-dihydroisoxazole-3-carboxylate (**11**)

Ethyl glyoxylate (39.6 mL of 50 wt% in PhMe, 200 mmol) was added to a stirring room temperature suspension of hydroxylamine hydrochloride (13.9 g, 200 mmol, 1.00 equiv.) in 9:1 acetonitrile/ water (162 mL). After ~5 min, triethylamine (27.7 mL, 200 mmol, 1.00 equiv.) was added dropwise over 30 min. The reaction was stirred for an additional 1 h and concentrated on a rotary evaporator under reduced pressure. The crude residue was extracted with Et₂O (3 × 100 mL) and the combined organic extracts washed with water (1 × 100 mL), brine (1 × 100 mL), dried over Na₂SO₄ and concentrated on a rotary evaporator to afford a clear, colorless oil as the intermediate oxime (22.2 g, 95% yield) that was used without further purification.

¹H NMR (300 MHz, CDCl₃): δ 7.56 (s, 1H), 4.32 (q, *J* = 7.0 Hz, 2H), 1.34 (t, *J* = 7.0 Hz, 3H). ¹³C NMR (75.5 MHz, CDCl₃): δ 162.4, 141.6, 61.8, 13.8.

Ethyl glyoxylate oxime (22.2 g, 189 mmol) was added to a solution of 1:1 ethyl vinyl ether/stock MeOH (300 mL). The mixture was cooled in an ice bath and PhI(OAc)₂ (71.5 g, 222 mmol, 1.2 equiv.) was added in portions, ensuring the internal temperature did not exceed 35 °C. The flask was flushed with argon and the reaction mixture was allowed to warm up over 18 h, at which point it was concentrated on a rotary evaporator under reduced pressure. The crude residue was purified by flash column chromatography (80:20 hexanes/EtOAc) to afford a pale-yellow oil as dihydroisoxazole **11** (23.6 g, 67% yield).

¹H NMR (300 MHz, CDCl₃): δ 5.72 (dd, *J* = 6.8, 2.0 Hz, 1H), 4.37 (q, *J*= 7.5 Hz, 2H), 3.91 (dq, *J* = 9.46, 7.12 Hz, 1H), 3.62 (dq, *J* = 9.46, 7.12 Hz, 1H), 3.26 (dd, *J* = 18.3, 6.8 Hz, 1H), 3.09 (dd, *J* = 18.5, 2.2 Hz, 1H), 2.18 (s, 3H), 1.38 (t, *J* = 6.0 Hz, 3H), 1.23 (t, *J* = 6.0 Hz, 3H). ¹³C NMR (75.5 MHz, CDCl₃): δ 160.1, 151.9, 104.9, 64.4, 62.1, 40.1, 14.9, 14.08. 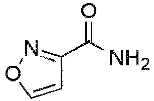 Isoxazole-3-carboxamide (**12**)

Anhydrous ammonia was bubbled through an ice-cold MeOH (35 mL) solution of dihydroisoxazole **11** (15.5 g, 82.93 mmol) in a round bottom flask. The mixture was allowed to warm to room temperature and after amide formation was deemed complete (TLC), NaOH pellets (3.32 g, 82.93 mmol, 1.00 equiv.) were added and the resultant stirred for 2 h. The reaction mixture was adjusted to pH ~7 by addition of aqueous 3 M HCl and extracted with EtOAc (3 × 75 mL). The combined organic extracts were washed with brine (1 × 75 mL), dried over Na₂SO₄ and concentrated on a rotary evaporator under reduced pressure to afford a white solid as carboxamide **12** (5.29 g, 57% yield) that was used without further purification.

¹H NMR (300 MHz, acetone-*d₆*): δ 8.87 (d, *J* = 1.8 Hz, 1H), 7.49 (br s, 1H), 7.11 (br s, 1H), 6.81 (d, *J* = 1.8 Hz, 1H).

¹³C NMR (75.5 MHz, acetone-*d₆*): δ 160.6, 138.9, 109.7, 107.2. 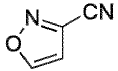 Isoxazole-3-carbonitrile (**9**)

Phosphorus oxychloride (2.52 mL, 27.1 mmol) was added dropwise to a room temperature pyridine (35 mL) solution of carboxamide **12** (2.03 g, 18.1 mmol) in a round bottom flask under argon. After stirring for 2 h, the mixture was cooled in an ice bath, adjusted to pH ~4 by addition of aqueous 3 M HCl and extracted with Et₂O (3 × 50 mL). The combined organic extracts were washed with aqueous 3 M HCl (1 × 50 mL), water (1 × 50 mL), brine (1 × 50 mL), dried over Na₂SO₄, and concentrated on a rotary evaporator under reduced pressure to afford a brown oil. The crude was purified by vacuum distillation afford a clear, colorless oil as nitrile **9** (1.26 g, 74% yield).

¹H NMR (300 MHz, CDCl₃) : δ 8.69 (d, *J* = 1.8 Hz, 1H), 6.76 (d, *J* = 1.8 Hz, 1H).

¹³C NMR (75.5 MHz, CDCl₃): δ 160.6, 138.9, 109.7, 107.2. 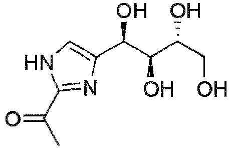 1-(4-((1*R*,2*S*,3*R*)-1,2,3,4-Tetrahydroxybutyl)-1*H*-imidazol-2-yl)ethan-1-one (**1**), also known as THI

*From glucosamine-HCl:* in a round bottom flask under argon, "*F1*", MeONa (3.00 mL of 3.0 M solution in MeOH, 9.00 mmol, 0.30 equiv.) was added by syringe to an ~10 °C MeOH (23 mL) solution of 2-ethoxyacrylonitrile (2.91 g, 30.0 mmol, 1.00 equiv.), which was allowed to stir for 8 h. In a second round bottom flask, "F2", equipped with an addition funnel and under argon, NaOAc (2.95 g, 36.0 mmol, 1.20 equiv.) was added to a room temperature MeOH (23 mL) suspension of glucosamine-HCl (7.76 g, 36.0 mmol, 1.20 equiv.). After stirring *F2* for 2 h, it was cooled to ~10 °C, the clear, colourless solution contained in *F1* was transferred to the F2 addition funnel and added to the contents of F2 dropwise over 15-20 min. The resultant was allowed to stir and warm up to room temperature over ~27 h, at which point it was cooled back to ~10 °C and a second portion of MeONa (6.5 mL of 3.0 M solution in MeOH, 19.5 mmol, 0.65 equiv.) was added to *F2*. After stirring for a further 24 h, water (35 mL) and HOAc (3.7 mL, 66.0 mmol, 2.20 equiv.) were added to the light yellow slurry and the mixture was heated at 60 °C for 3 h, during which time darkening to brown occurred. Heating was ceased and the reaction mixture concentrated on a rotary evaporator (bath temperature 45 °C) under reduced pressure to ~15% volume, then cooled in an ice bath for ~ 1.5 h and the solids were collected by suction filtration, washing with minimal ice-cold water (2 × 25 mL), to afford an off-white powder as THI (4.23 g, 61% yield).

¹H NMR (300 MHz, D₂O + 1 M HCl): δ 7.51 and 7.23 (s, 1H), 5.13 and 5.03 (d, *J* = 1.5 Hz, 1H), 3.75- 3.50 (m, 4H), 2.58 (s, 3H).

¹³C NMR (75.5 MHz, D₂O + 1 M HCl): δ 184.6, 139.1, 137.6, 119.0, 72.5, 70.4, 64.6, 62.7, 26.2.

*From fructosamine-HCl:* in a round bottom flask under argon, MeONa (5.00 mL of 3.0 M solution in MeOH, 15.0 mmol, 0.50 equiv.) was added by syringe to a room temperature MeOH (31 mL) solution of 2-ethoxyacrylonitrile (3.00 g, 30.9 mmol, 1.03 equiv.). After stirring for 5 h, fructosamine-HOAc (7.18 g, 30.0 mmol, 1.00 equiv.) was added in one portion and stirring continued for 4 h, during which time a very thick white mixture developed. Then, a second portion of MeONa (6.0 mL of 3.0 M solution in MeOH, 18.0 mmol, 0.60 equiv.) was added to the reaction mixture. After stirring for a further 20 h, water (31 mL) and HOAc (3.40 mL, 60.0 mmol, 2.00 equiv.) were added and the mixture was heated at 60 °C for 1.5 h, during which time darkening to brown occurred. Heating was ceased and the reaction mixture concentrated on a rotary evaporator (bath temperature 45 °C) under reduced pressure to -15% volume, then cooled in an ice bath for ~1.5 h and the solids were collected by suction filtration, washing with minimal ice-cold water (2 × 20 mL), to afford an off-white powder as THI (5.15 g, 75% yield). 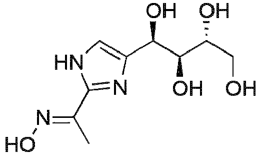 1-(4-((1*R*,2*S*,3*R*)-1,2,3,4-Tetrahydroxybutyl)-1*H*-imidazol-2-yl)ethan-1-one oxime (**2**), also known as LX2931 - JZ-26-007, 23-101

A MeOH (60 mL) suspension of THI (6.91 g, 30.0 mmol), hydroxylamine hydrochloride (3.13 g, 45.0 mmol, 1.50 equiv.) and NaOAc (3.69 g, 45.0 mmol, 1.50 equiv.) in around bottom flask equipped with a condenser and under argon was heated at reflux. After 2.5 h, the mixture was cooled to room temperature, HCl solution (22.5 mL of 4 M solution in dioxane, 90.0 mmol, 3.00 equiv.) was added and heating at reflux was resumed for another 2.5 h. The reaction mixture was cooled removed from heat, diluted with MeOH (180 mL, 3 volumes), cooled in an ice bath for 1.5 h and the precipitated solids were removed by filtration through Celite^{®}. The yellow filtrate was concentrated on a rotary evaporator under reduced pressure to ~1 volume (60 mL), then water (180 mL, 3 volumes) was added and the mixture was concentrated again on a rotary evaporator to 0.5-1 volume. Aqueous 6 M NaOH was added to the concentrate to adjust to pH 6-8 and the yellowish solution allowed to stand at room temperature until crystallization began, at which point it was cooled in an ice bath for ~2 h. The solids were collected, washed with ice-cold water (2× 30 mL), and dried to afford fine, white needles as LX2931 (6.97 g, 95% yield).

¹H NMR (300 MHz, D₂O + 1 M HCl): δ 7.32 (s, 3H), 5.06 (s, 2H), 3.78-3.47 (m, 4H), 2.16 (s, 3H), 3.50 (m, 4H).

¹³C NMR (75.5 MHz, D₂O + 1 M HCl): δ 143.8, 141.0, 135.0, 116.9, 72.5, 70.5, 64.4, 62.7, 10.4. 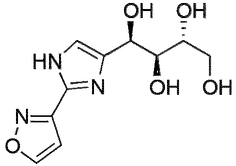 (1R,2S,3R)-1-(2-(isoxazol-3-yl)-1H-imidazol-4-yl)butane-1,2,3,4-tetraol (**3**), also known as LX2932

*From glucosamine-HCl:* In a round bottom flask under argon, "*F1*", MeONa (0.35 mL of 5.0 M solution in MeOH, 1.75 mmol, 0.30 equiv.) was added by syringe to an ~10 °C MeOH (4 mL) solution of nitrile **9** (508 mg, 5.40 mmol, 1.00 equiv.), which was allowed to stir for 7 h. In a second round bottom flask under argon, "F2", NaOAc (531 mg, 6.42 mmol, 1.20 equiv.) was added to a room temperature MeOH (4 mL) suspension of glucosamine-HCl (1.40 g, 6.48 mmol, 1.20 equiv.). After stirring *F2* for 1 h, the clear, colourless solution contained in *F1* was added to *F2* by syringe over 10 min. The resultant was allowed to stir for ~24 h, at which point it was cooled back to ~10 °C and a second portion of MeONa (0.68 mL of 5.0 M solution in MeOH, 3.38 mmol, 0.63 equiv.) was added to *F2.* After stirring for a further 5 h while warming back to room temperature, water (8 mL) and HOAc (0.62 mL, 11.9 mmol, 2.20 equiv.) were added, the reaction mixture was stirred 10 min, then concentrated on a rotary evaporator (bath temperature 45 °C) under reduced pressure to ~25% volume, then cooled in an ice bath for -1.5 h and the solids were collected by suction filtration, washing with minimal ice-cold water (2 × 10 mL), to afford a white powder as LX2932 (745 mg, 54% yield).

¹H NMR (300 MHz, D₂O + 1 M HCl): δ 8.64 (d, *J* = 1.8 Hz, 1H), 7.34 (d, *J* = 0.9 Hz, 1H), 6.84 (d, *J* = 2.2 Hz, 1H), 4.99 (dd, *J* = 1.8, 0.9 Hz, 1H), 3.70-3.32 (m, 4H).

¹³C NMR (75.5 MHz, D₂O + 1 M HCl): δ 162.2, 148.6, 136.1, 134.0, 117.7, 103.5, 72.5, 70.4, 64.4, 62.64.

*From fructosamine-HCl:* in a round bottom flask under argon, MeONa (1.79 mL of 3.0 M solution in MeOH, 15.0 mmol, 0.50 equiv.) was added by syringe to a room temperature MeOH (31 mL) solution of 2-ethoxyacrylonitrile (3.00 g, 30.9 mmol, 1.03 equiv.). After stirring for 5 h, fructosamine-HOAc (7.18 g, 30.0 mmol, 1.00 equiv.) was added in one portion and stirring continued for 4 h, during which time a very thick white mixture developed. Then, a second portion of MeONa (6.0 mL of 3.0 M solution in MeOH, 18.0 mmol, 0.60 equiv.) was added to the reaction mixture. After stirring for a further 20 h, water (31 mL) and HOAc (3.4 mL, 60.0 mmol, 2.00 equiv.) were added and the mixture was heated at 60 °C for 1.5 h, during which time darkening to brown occurred. Heating was ceased and the reaction mixture concentrated on a rotary evaporator (bath temperature 45 °C) under reduced pressure to -15% volume, then cooled in an ice bath for -1.5 h and the solids were collected by suction filtration, washing with minimal ice-cold water (2 × 20 mL), to afford an off-white powder as LX2932 (5.15 g, 75% yield). 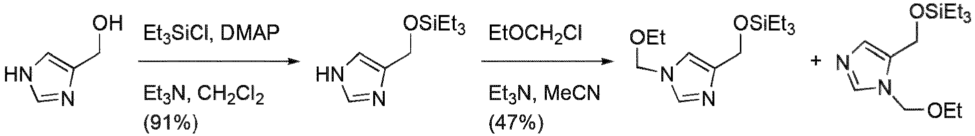 1-Ethoxymethyl-4-((triethylsilyloxy)methyl)imidazole and 1-Ethoxymethyl-5-((triethylsilyloxy)methyl)imidazole (**13**) - JZ-26-111, 107, 080, 073, 071; JZ-26-105, 101, 079, 070, 069

Neat chlorotriethylsilane (11.0 mL, 66.0 mmol, 1.10 equiv.) was added slowly to a ~10 °C CH₂Cl₂ (50 mL) solution of 4-hydroxymethylimidazole (5.89 g, 60.0 mmol, 1.00 equiv.) and triethylamine (27.4 mL, 198 mmol, 3.30 equiv.) in a round bottom flask under argon, which resulted in an exothermic formation of a thick white precipitate. After stirring for 16 h, the reaction mixture was diluted with CH₂Cl₂ (200 mL), washed with water (2 × 200 mL), dried over Na₂SO₄, and concentrated on a rotary evaporator under reduced pressure. The crude pale yellow oil was filtered through a plug of silica gel, eluting with 75:25→100:0 EtOAc/hexanes, and the filtrate concentrated on a rotary evaporator under reduced pressure to afford a clear, colourless oil as the intermediate *O*-triethylsilyl hydroxymethylimidazole (11.6 g, 91% yield). R_{f} 0.12 (silica gel, 90:10 EtOAc/hexanes)

¹H NMR (300 MHz, CDCl₃): δ 9.78 (br s, 1H), 7.61 (s, 1H), 6.97 (s, 1H), 4.74 (s, 2H), 0.97 (t, *J* = 8.0 Hz, 9H), 0.65 (q, *J* = 7.9 Hz, 6H).

Chloromethyl ethyl ether (3.94 mL, 42.6 mmol, 1.80 equiv.) was added slowly to a room temperature MeCN (25 mL) solution of the above silyl ether (5.04 g, 23.6 mmol, 1.00 equiv.) and triethylamine (6.55 mL, 47.3 mmol, 2.00 equiv.) in a round bottom flask equipped with a condenser and under argon, which was was then heated at 60 °C. After 3 h, the reaction mixture was allowed to cool to room temperature, water (75 mL) was added and the mixture extracted with EtOAc (2 × 100 mL). The combined organic extracts were washed with water (1 × 100 mL), brine (1 × 100 mL), dried over Na₂SO₄ and concentrated on a rotary evaporator under reduced pressure. The crude yellow oil was purified by flash column chromatography on silica gel (50:50 to 75:25 to 100:0 EtOAc/hexanes) to afford a pale yellow oil as an ~1:1 regioisomeric mixture of protected imidazoles **13** (3.02 g, 47% yield).
R_{f} 0.28 (silica gel, 90:10 hexanes/EtOAc)
¹H NMR (300 MHz, CDCl₃): 7.54 (br d, *J* = 8.1 Hz, 2H), 6.98 (br d, *J* = 11.8 Hz, 2H), 5.39 (s, 2H), 5.25 (s, 2H), 4.72 (br d, *J* = 5.2 Hz, 4H), 3.53-3.40 (m, 4H), 1.19 (br t, *J* = 7.2 Hz, 6H), 0.97 (app q, *J* = 8.4 Hz, 18H), 0.73-0.56 (m, 12H) 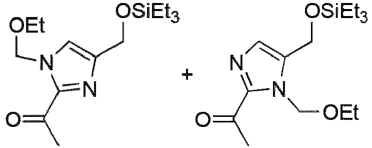
1-(1-(Ethoxymethyl)-4-(((triethylsilyl)oxy)methyl)-1*H*-imidazol-2-yl)ethan-1-one and 1-(1-(Ethoxymethyl)-5-(((triethylsilyl)oxy)methyl)-1*H*-imidazol-2-yl)ethan-1-one (**14**) - JZ-26-113, XX
*n*-BuLi (10.7 mL of 2.20 M solution in hexanes, 23.5 mmol, 1.50 equiv.) was added to a -78 °C THF (35 mL) solution of protected imidazoles **13** (4.24 g, 15.6 mmol, 1.00 equiv.) in a round bottom flask under argon, which produced a pale yellow color. After stirring for 1 h at -78 °C, neat *N*-methoxy-*N*-methylacetamide (3.33 mL, 31.3 mmol, 2.00 equiv.) was added and the reaction mixture was allowed to warm to room temperature over 16 h, at which point it was quenched by addition of saturated aqueous NH₄Cl (100 mL) and extracted with EtOAc (2 × 100 mL). The combined organics were washed with water (1 × 100 mL), brine (1 × 100 mL), dried over Na₂SO₄ and concentrated on a rotary evaporator under reduced pressure to provide a yellow oil. The crude was filtered through a plug of silica gel, eluting with 80:20 hexanes/EtOAc, to afford a pale yellow oil as a ~1:1 regioisomeric mixture of acylimidazoles **14** (4.44 g, 91%).
R_{f} 0.59 (silica, 60:40 EtOAc/hexanes)
¹H NMR (300 MHz, CDCl₃): δ 7.26 (s, 1H), 7.10 (s, 1H), 5.95 (s, 2H), 5.78 (s, 2H), 4.80 (s, 2H), 4.75 (s, 2H), 3.54 (app quint, *J* = 7.3 Hz, 4H), 2.69 (s, 3H), 2.66 (s, 3H), 1.25-1.12 (app m, 6H), 0.98 (app q, *J* = 7.8 Hz, 18H), 0.66 (app quint, *J* = 7.9 Hz, 12H). 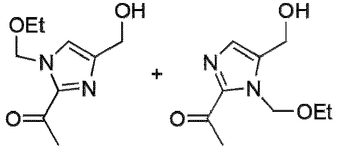
1-(1-(Ethoxymethyl)-4-(hydroxymethyl)-1*H*-imidazol-2-yl)ethan-1-one and 1-(1-(Ethoxymethyl)-5-(hydroxymethyl)-1*H*-imidazol-2-yl)ethan-1-one (**15**) - JZ-26-116, 115, 099, 089, 087, 085
Solid K₂CO₃ (3.87 g, 28.0 mmol, 2.00 equiv.) was added to a room temperature MeOH (35 mL) solution of silyl ethers **14** (4.37 g) in a round bottom flask under argon. After stirring the slightly yellow reaction mixture for 1.5 h, it was diluted with EtOAc and filtered through Celite^{®}. Silica gel was added to the filtrate and the mixture concentrated to dryness on a rotary evaporator under reduced pressure. The loaded silica gel was added to the top of a pad of silica gel and eluted with 98:2 EtOAc/MeOH. The filtrate was concentrated on a rotary evaporator under reduced pressure to afford an off-white solid as a ~1:1 regioisomeric mixture of hydroxymethylimidazoles **15** (2.59 g, 93% yield).

*Note:* This material could be recrystallized from 1:1 *t*-BuOMe/hexanes at -20 °C to produce samples of analytical quality, but this was found unnecessary. Upon completion of the reaction, if the mixture was handled to any significant degree before adding an acidic agent (silica gel, as described above), colours ranging from orange to pink that eventually gave way to brown and black in some cases were noted that coincided with sometimes significant material losses. This is believed to be due to oxidation (from atmospheric oxygen) of unquenched enolates or related isomers.

R_{f} 0.24 and 0.14 (silica, 75:25 EtOAc/hexanes)
¹H NMR (300 MHz, DMSO-*d₆*): δ 7.50 (s, 1H), 7.10 (s, 1H), 5.79 (s, 2H), 5.68 (s, 2H), 5.37 (t, *J* = 5.5 Hz, 1H), 5.11 (t, *J* = 5.6 Hz, 1H), 4.57 (d, *J* = 5.6 Hz, 2H), 4.42 (d, *J* = 5.6 Hz, 2H), 3.51-3.39 (app m, 4H), 2.56 (s, 3H), 2.54 (s, 3H), 1.13-1.00 (app m, 6H). 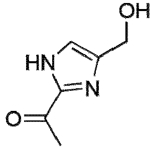 1-(4-(Hydroxymethyl)-1*H*-imidazol-2-yl)ethan-1-one (**4**), also known as A6670 - JZ-26-123, 119, 117, 103

*N*-protected imidazoles **15** (892 mg, 4.50 mmol, 1.00 equiv.) were dissolved in aqueous 1.25 M HCl (22 mL) in a round bottom flask under argon equipped with a condenser and under argon, then heated at 70 °C. After 16 h, the pale yellow reaction mixture was cooled to room temperature and adjusted to pH ~6 by addition of aqueous 3 M NaOH, then concentrated to dryness on a rotary evaporator (bath temperature 45 °C) under reduced pressure. *i*-PrOH and activated carbon were added to the crude solids and the mixture warmed gently, then filtered through Celite^{®}. This material was filtered through a plug of silica gel, eluting with 95:5 EtOAc/MeOH, to afford a white solid as A6670 (600 mg, 95% yield)

*Note:* This material could be recrystallized from 1:1 *t*-BuOMe/*i*-PrOH at -20 °C, if desired. Again, care should be taken with the pH of this mixture after the reaction is complete as any processing of material that had experienced pH>6 tended to become coloured. Removal of these coloured impurities by treatment with activated carbon or recrystallization was not always successful. It is believed that this behavior is also due to oxidation (from atmospheric oxygen) of enolates or related isomers.

R_{f} 0.40 (silica, 95:5 EtOAc/MeOH)
¹H NMR (300 MHz, CD₃OD): δ 7.21 (br s, 1H), 4.62 (s, 2H), 2.55 (s, 3H).

¹³C NMR (5.5 MHz, CD₃OD): δ 189.3, 179.0, 146.5, 141.8, 123.7, 56.5. 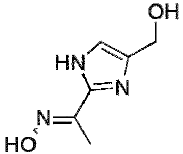 1-(4-(Hydroxymethyl)-1*H*-imidazol-2-yl)ethan-1-one oxime (**5**) - JZ-26-125, 121

Solid hydroxylamine hydrochloride (334 mg, 4.80 mmol, 1.20 equiv.) and NaOAc (656 mg, 8.00 mmol, 2.00 equiv.) were added to a room temperature water (10 mL) solution of A6670 (560 mg, 4.00 mmol, 1.00 equiv.) in a round bottom flask under argon and the resulting pale yellow solution heated at 50 °C. After 16 h, the reaction mixture was concentrated on a rotary evaporator (bath temperature 45 °C) under reduced pressure to obtain the crude as off-white solids. The crude was suspended in 4:1 EtOAc/MeOH, filtered through Celite^{®} and concentrated on a rotary evaporator under reduced pressure to leave a yellow oil, which was crystallized from water at 0 °C in three batches to afford white crystals as a ~5.5:1 *E*/*Z* or *Z*/*E* (isomers not assigned) isomeric mixture of oxime **5** (434 mg, 70% yield).

*Note:* This material could also be recrystallized from MeOH/EtOAc/t-BuOMe at -20 °C.

¹H NMR (300 MHz, CD₃OD): δ 7.15 and 7.00 (s, 1H), 4.63 and 4.57 (s, 2H), 2.26 and 2.23 (s, 3H).

¹³C NMR (75.5 MHz, CD₃OD): δ 148.2, 145.6, 144.1, 141.0, 57.6 (br), 17.9, 10.6. 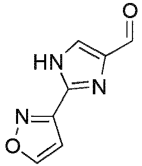 2-(Isoxazol-3-yl)-1*H*-imidazole-4-carbaldehyde (**16**)

Solid NaIO₄ (40 mg, 1.89 mmol, 4.70 equiv.) was added to a 85:10:5 H₂O/MeOH/HOAc solution (21 mL) of LX2932 (93 mg, 0.40 mmol, 1.00 equiv.) in a round bottom flask open to air. After 3 h, the reaction was quenched by addition of saturated aqueous NaHCO₃ and extracted with EtOAc (3 × 10 mL). The combined organic extracts were washed with brine (1 × 10 mL), dried over Na₂SO₄ and concentrated on a rotary evaporator under reduced pressure. The crude was filtered through a short plug of silica gel, eluting with EtOAc, to afford a white solid as aldehyde **16** (44 mg, 67% yield).

¹H NMR (300 MHz, acetone-*d₆*): δ 9.90 (s, 1H), 8.91 (d, *J* = 1.8 Hz, 1H), 8.09 (s, 1H), 7.03 (d, *J* = 1.8 Hz, 1H).

¹³C NMR (75.5 MHz, acetone-*d₆*): δ 184.6, 161.4, 155.0, 142.3, 140.4, 129.0, 104.0. 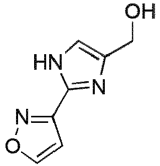 (2-(Isoxazol-3-yl)-1*H*-imidazl-4-yl)methanol (**6**)

Solid NaBH₄ (9 mg, 0.24 mmol, 1.50 equiv.) was added to a room temperature MeOH (1.5 mL) solution of aldehyde **16** (26 mg, 0.16 mmol, 1.00 equiv.) in a round bottom flask under argon. After 3 h, the reaction was quenched by addition of saturated aqueous NH₄Cl and extracted with 2:1 CH₂Cl₂/*i*-PrOH (3 × 5 mL). The combined organic extracts were dried over Na₂SO₄ and concentrated on a rotary evaporator under reduced pressure. The crude was dissolved in MeCN/MeOH and washed with hexanes (3 × 3 mL) to remove greasy contaminants. The MeCN/MeOH layer was evaporated to afford a white powder as hydroxymethylimidazole **6** (20 mg, 73% yield).

¹H NMR (300 MHz, acetone-*d₆*): δ 8.79 (d, *J* = 1.8 Hz, 1H), 7.16 (s, 1H), 6.92 (d, *J* = 1.4 Hz, 1H), 5.45 (br s, 1H), 4.62 (d, *J* = 0.7 Hz, 2H).

¹³C NMR (75.5 MHz, acetone-*d₆*): δ 160.6, 155.6, 141.5, 137.4, 120.7, 103.7, 57.6. 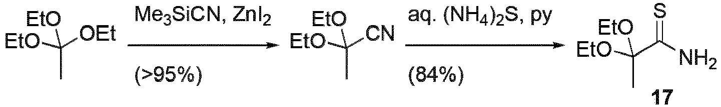 2,2-Diethoxypropanethioamide (**17**) - JZ-26-021, 23-171, 165, 164, 163; 26-022, 23-169, 160, 069

Triethyl orthoacetate (12.4 mL, 68.1 mmol, 1.00 equiv.), then zinc iodide (108 mg, 0.34 mmol, 0.5 mol%), was added to a round bottom flask containing trimethylsilyl cyanide (7.09 g, 71.5 mmol, 1.05 equiv.) under argon and maintained at 10-15 °C with a water bath. After the addition was complete, the water bath was removed and the reaction mixture allowed to stir for a further 5 h, at which point it was quenched by addition of saturated aqueous NaHCO₃. The mixture was extracted with EtzO (3 × 50 mL), then the combined organic extracts washed with saturated aqueous NaHCO₃ (1 × 50 mL), dried over Na₂SO₄, filtered and concentrated on a rotary evaporator under reduced pressure to afford a pale yellow oil as 2,2-diethoxypropionitrile (9.34 g, 96% yield) that was used without further purification.

¹H NMR (300 MHz, CDCl₃): δ 3.68 (m, 4H), 1.46 (s, 3H), 1.27 (t, *J* = 7.0 Hz, 6H)

¹³C NMR (300 MHz, CDCl₃): δ 117.1, 96.0, 59.8, 24.7, 14.8.

Ammonium sulfide (11.6 mL of 42 wt% solution in water, 71.4 mmol, 1.10 equiv.) was added to a stirring room temperature pyridine (65 mL) solution of 2,2-diethoxypropionitrile (9.29 g, 64.9 mmol, 1.00 equiv.) in a round bottom flask under argon. After 18 h, water (150 mL) was added and the mixture was extracted with EtOAc (3 × 100 mL). The combined organic extracts were washed with water (1 × 100 mL), brine, dried over Na₂SO₄, filtered and concentrated on a rotary evaporator under reduced pressure to afford an off-white powder as thioamide **17** (9.68 g, 84% yield) that was used without further purification.

¹H NMR (300 MHz, CDCl₃): δ 8.29 (br s, 1H), 7.95 (br s, 1H), 3.65-3.42 (m, 4H), 1.68 (s, 3H), 1.24 (t, *J* = 6.0 Hz, 6H).

¹³C NMR (300 MHz, CDCl₃): δ 206.6, 103.0, 58.0, 24.7, 15.2. 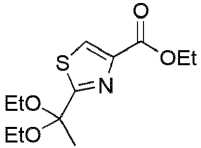 Ethyl 2-(1,1-diethoxyethyl)thiazole-4-carboxylate (**18**)

Ethyl glyoxylate (0.30 mL, 2.41 mmol, 1.30 equiv.) was added to an ice-cold THF (9 mL) mixture of thioamide **17** (329 mg, 1.86 mmol, 1.00 equiv.) and NaHCO₃ (1.56 g, 18.6 mmol, 10.0 equiv.) in a round bottom flask under argon, which was allowed to warm up over 18 h. The reaction mixture was then filtered through Celite^{®}, washing with THF, and concentrated on a rotary evaporator under reduced pressure to provide the intermediate thiazoline. The crude intermediate was suspended in THF (9 mL) under argon, cooled in an ice bath and pyridine (1.5 mL,18.6 mmol, 10.0 equiv.) was added. Trifluoroacetic anhydride (1.11 mL, 7.98 mmol, 4.30 equiv.) was added to the reaction mixture over 30 min and the resultant stirred for a further 4 h in an ice bath. While still in an ice bath, triethylamine (0.52 mL, 3.71 mmol, 3.71 equiv.) was added dropwise, then the reaction mixture left to stir for 1 h, at which point saturated aqueous NaHCO₃ was added. The mixture was extracted with EtOAc (3 × 10 mL). The combined organic extracts were washed with brine (1 × 10 mL), dried over Na₂SO₄ and concentrated under reduced pressure. The crude was purified by flash column chromatography (74:25:1 hexanes/EtOAc/Et₃N) to afford a pale yellow oil that solidified on standing as thiazole **18** (496 mg, 97% yield).

¹H NMR (300 MHz, acetone-*d₆*): δ 7.32 (t, *J* = 1.1 Hz, 1H), 4.68 (dd, *J* = 5.86, 1.1 Hz, 2H), 4.31 (t, *J* = 5.9 Hz, 1H), 3.65-3.51 (m, 2H), 3.42-3.30 (m, 2H), 1.66 (s, 3H), 1.15 (t, *J* = 7.0 Hz, 6H).

¹³C NMR (75.5 MHz, acetone-*d₆*): δ 173.1, 159.1, 115.5, 101.2, 61.6, 57.8, 25.3,15.6. 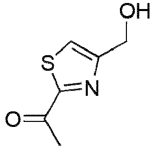 2-Acetyl-4-hydroxymethylthiazole (7)

A THF (3 mL) solution of ester **18** (481 mg, 1.76 mmol, 1.00 equiv.) was added dropwise to an ice-cold THF (3 mL) suspension of LiAlH₄ (80 mg, 2.11 mmol, 1.20 equiv.) in a round bottom flask under argon. After stirring for 30 min, the ice bath was removed and stirring continued for an additional 30 min, at which point the reaction mixture cooled in an ice bath and quenched by addition of Na₂SO₄·10 H₂O. The reaction mixture was stirred at room temperature until gas evolution ceased (~3 h), then filtered through Celite^{®}, washing with EtOAc, and the filtrate concentrated on a rotary evaporator under reduced pressure to afford an off-white solid. The crude was recrystallized from EtOAc/hexanes to afford white needles as hydroxymethylthiazole 7 (286 mg, 70% yield).

¹H NMR (300 MHz, acetone-*d₆*): δ 7.82 (t, *J* = 1.0 Hz, 1H), 4.79 (dd, *J* = 5.7, 0.9 Hz, 2H), 4.57 (t, *J* = 5.8 Hz, 1H) 2.62 (s, 3H).

¹³C NMR (75.5 MHz, acetone-*d₆*): δ 191.9, 167.5, 161.3, 122.6, 61.2, 25.9.

### Example 2

The following example is not according to the invention and is present for illustration purposes only.

The evaluation of the thermodynamic binding parameters of different sphingosine-1-phosphate inhibitors has been carried out. The assay has been done with the phosphorylated compounds which are the actual S1PL inhibitors in-vivo.

Crystal structure of S1PL in the PDB (4Q6R) was used to perform the docking experiments. Docking was made with GOLD (Protein-Ligand docking using Genetic Algorithm) using the CHEMPLP scoring function (Protein-Ligand ANT System).

Poses were optimized and rescored with ChemScore to obtain ΔG binding energies. CHARMM force field was used to estimate the total binding and interaction energies of the rescored poses.

### Example 2-1:

Phosphorylated THI metabolite and derivatives

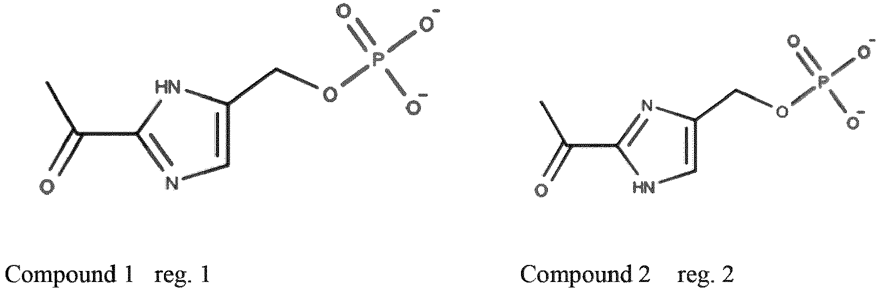

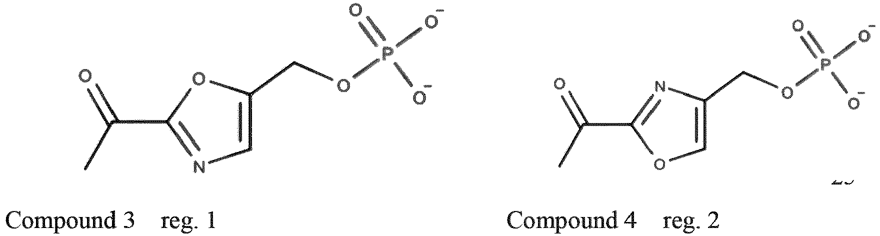

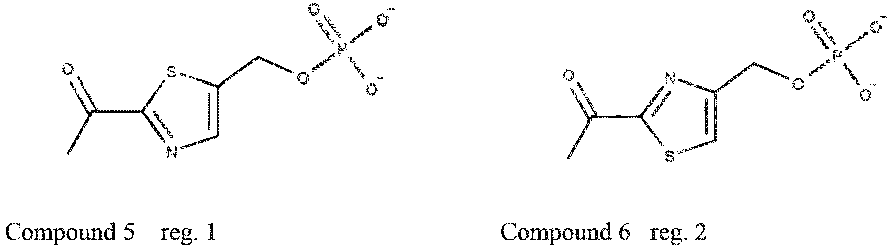

**Table 2: Thermodynamic parameters describing the binding of human sphingosine-1-phosphate Lyase inhibitors to the catalytic site of the enzyme.**

| Compounds | Total Interaction Energy Kcal/mol | Total VDW Interaction energy Kcal/mol | Total Electrostatic Interaction Energy Kcal/mol | ΔG Binging Energy Kcal/mol | Total Binging Energy Kcal/mol CHARMM* |
|---|---|---|---|---|---|
| S1P | -276.01532 | 9.39604 | -285.41136 | -31.254 | -504.836 |
| Compound 1 | -284.26823 | -5.66954 | -278.59869 | -28.13 | -188.55 |
| Compound 2 | -291.67197 | -2.81322 | -288.85875 | -28.05 | -186.28 |
| Compound 3 | -278.17480 | 3.30884 | -281.48364 | -28.09 | -706.22 |
| Compound 4 | -237,43263 | -0.6689 | -236.76875 | -15.10 | -695.54 |
| Compound 5 | -108.66511 | 177.49551 | -286.16062 | -30.55 | -710.18 |
| Compound 6 | -227.89978 | -4.27660 | -223.62118 | -11.99 | -701.26 |

| | | | | | |
|---|---|---|---|---|---|
| S1P: Sphingosine-1-phosphate. VDW: Van Der Walls. *Absolute binding free energy | | | | | |

**Sphingosine-1-Phosphate (S1P):** S1P displays a high binding energy mainly coming from electrostatic interaction. A slight energetic penalty comes from the Van de Walls repulsion of about 9 Kcal/mol.

**Phosphorylated compounds 1 and 2 (Tautomer 1 and 2):** Tautomers correspond to the two possible position of the basic nitrogen of the imidazole ring. The phosphorylated forms of the compounds 1 and 2 display a strong binding energy mainly due to electrostatic interaction. The position of the nitrogen of the imidazole ring has a marginal impact on the binding. For both tautomers, the total interaction energy is close to the one of S1P. However, the total binding energy as calculated with all thermodynamics parameters provided by the force field CHARMM is much lower than the one of S1P (188 vs 504).

**Phosphorylated compounds 3 and 4 (oxazole ring regioisomers 1 and 2):** These compounds display a high binding energy mainly due to electrostatic interaction. The values of the total interaction energy and ΔG are close to those observed with the imidazole ring (THI). However, the total binding energy as calculated by CHARMM is much higher than the one observed for the phosphorylated compounds 3 and 4 and higher than the one of S1P (-706 vs -504).

**Phosphorylated compounds 5 and 6 (thiazole ring regioisomer 1 and 2):** These compounds displays a high binding energy mainly due to electrostatic interaction. The values of the total interaction energy is markedly lower than those of the imidazole and oxazole containing molecules, due a marked energetic penalty (VdW: +177). ΔG value is close to those observed with the imidazole ring and oxazole containing molecule. However, as seen with the oxazole molecule, the total binding energy as calculated by CHARMM is much higher than the one observed for the phosphorylated compounds 1 and 2 and higher than the one of S1P (-710 vs 504).

### Example 2-2:

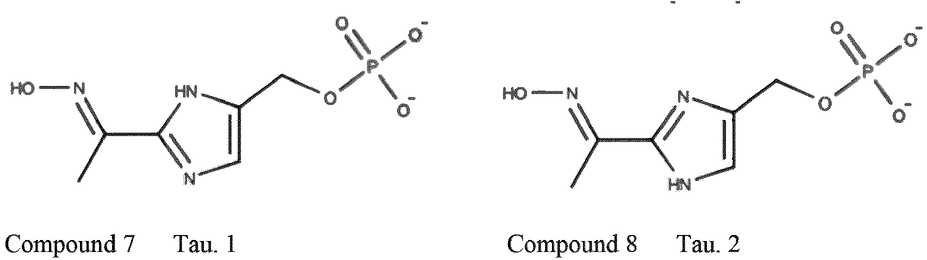

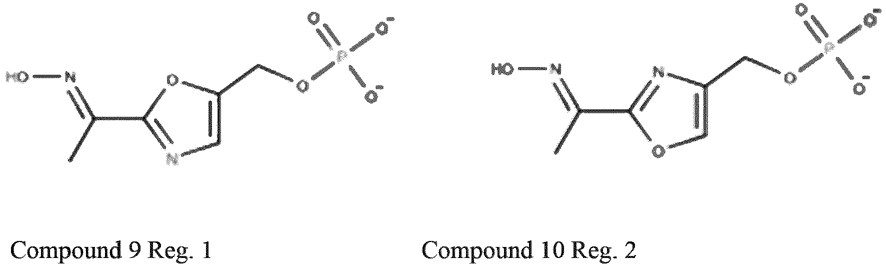

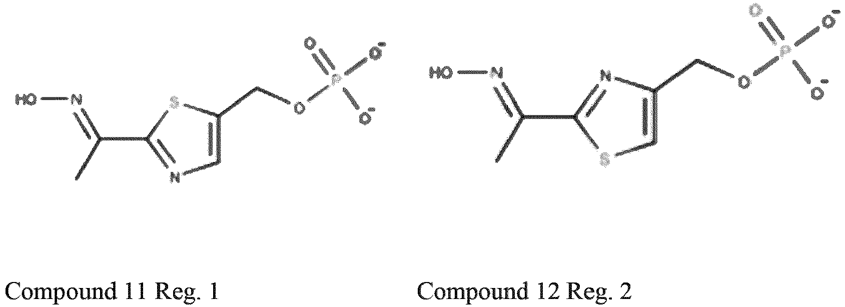

**Table 3: Thermodynamic parameters describing the binding of human sphingosine-1-phosphate Lyase inhibitors LX2931 and derivatives to the catalytic site of the enzyme.**

| Compounds | Total Interaction Energy Kcal/mol | Total VDW Interaction energy Kcal/mol | Total Electrostatic Interaction Energy Kcal/mol | ΔG Binging Energy Kcal/mol | Total Binging Energy Kcal/mol CHARMM* |
|---|---|---|---|---|---|
| S1P | -276.01532 | 9.39604 | -285.41136 | -31.25 | -504.836 |
| Compound 7 | -267.35952 | 5.62673 | -272.98626 | -27.92 | -192.63 |
| Compound 8 | -247.50954 | 28.09285 | -275.60239 | -27.10 | -186.75 |
| Compound 9 | -311.66049 | -3.49726 | -308.16333 | -15.45 | -717.80 |
| Compound 10 | -234.22074 | -5.24975 | -228.97099 | -17.01 | -725.34 |
| Compound 11 | -255.08918 | -6.47355 | -248.61563 | -18.93 | -725.34 |
| Compound 12 | -327.26240 | -3.97969 | -323.28271 | -15.55 | -717.42 |

| | | | | | |
|---|---|---|---|---|---|
| S1P: Sphingosine-1-phosphate. VDW: Van Der Walls. *Absolute binding free energy | | | | | |

**Phosphorylated compounds 7 and 8 (Tautomer 1 and 2):** Tautomers correspond to the two possible position of the basic nitrogen of the imidazole ring. The phosphorylated forms of these compounds display a strong binding energy mainly due to electrostatic interaction. For studied tautomers, the total interaction energy is close to the one of S1P. However, the total binding energy as calculated with all thermodynamics parameters provided by the force field CHARMM is much lower than the one of S1P (187 vs 504).

**Phosphorylated compounds 9 and 10 (oxazole ring regioisomer 1 and 2):** These compounds display as all phosphorylated compounds a high binding energy mainly due to electrostatic interaction. The total interaction energy values are close or higher to those observed with the imidazole ring. However, the total binding energy as calculated by CHARMM is much higher than the one observed for the phosphorylated imidazole containing compounds and higher than the one of S1P (-717 and -725 vs -504).

**Phosphorylated compounds 11 and 12 (thiazole ring regioisomer 1 and 2):** These compounds display a high binding energy mainly due to electrostatic interaction. The values of the total interaction energy is markedly are close to those observed with the oxazole containing compounds. ΔG value is close to those observed with oxazole containing compounds but lower compared to those displaying by the imidazole ring. However, as seen with the oxazole containing compounds, the total binding energy as calculated by CHARMM is much higher than the one observed for the phosphorylated imidazole containing compounds and higher than the one of S1P (-725 and -717 vs 504).

### Example 2-3:

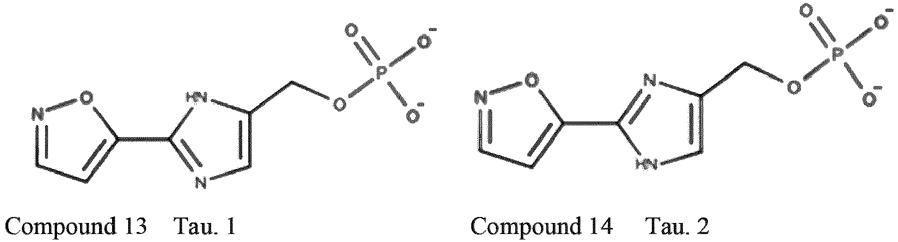

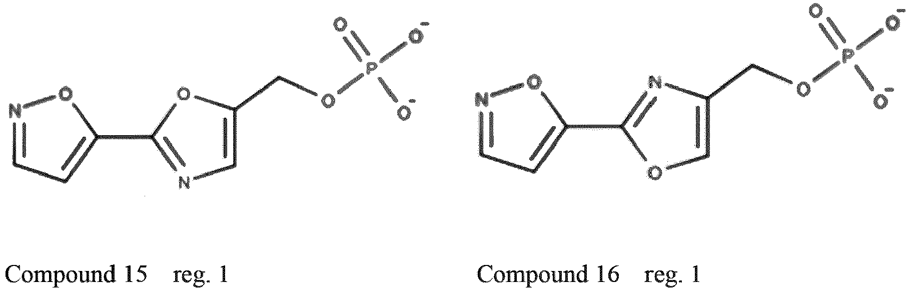

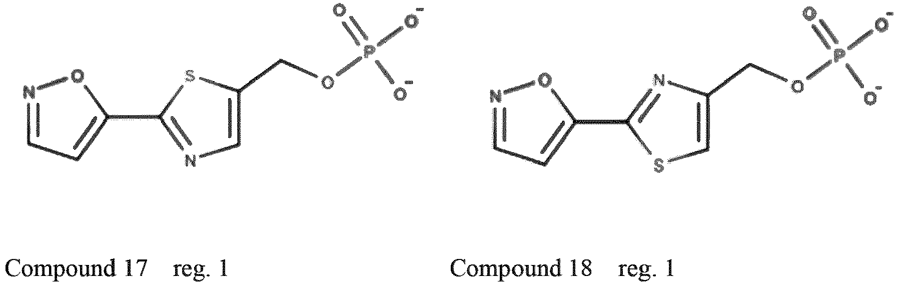

**Table 4: Thermodynamic parameters describing the binding of human sphingosine-1-phosphate Lyase inhibitors LX2932 and derivatives to the catalytic site of the enzyme.**

| Compounds | Total Interaction Energy Kcal/mol | Total VDW Interaction energy Kcal/mol | Total Electrostatic Interaction Energy Kcal/mol | ΔG Binging Energy Kcal/mol | Total Binging Energy Kcal/mol CHARMM* |
|---|---|---|---|---|---|
| S1P | -276.01532 | 9.39604 | -285.41136 | -31.25 | -504.836 |
| Compound 13 | -229.67669 | -4.61683 | -225.05986 | -23.21 | -754.60 |
| Compound 14 | -295.00186 | -1.84194 | -293.15992 | -23.59 | -739.17 |
| Compound 15 | -235.52535 | 7.53849 | -243.06384 | -18.14 | -697.66 |
| Compound 16 | -240.39398 | -7.74407 | -232.64991 | -17.90 | -702.19 |
| Compound 17 | -229.90385 | -5.49813 | -224.40571 | -16.78 | -696.31 |
| Compound 18 | -317.02957 | -4.30811 | -312.72146 | -21.34 | -712.67 |

| | | | | | |
|---|---|---|---|---|---|
| S1P: Sphingosine-1-phosphate. VDW: Van Der Walls. *Absolute binding free energy | | | | | |

**Phosphorylated compounds 13 and 14 (Tautomer 1 and 2):** Tautomers correspond to the two possible position of the basic nitrogen of the imidazole ring. The phosphorylated forms of these compounds display a strong binding energy mainly due to electrostatic interaction. For studied tautomers, the total interaction energy is close to the one of S1P. In contrast with the other imidazole containing ring, the total binding energy as calculated with all thermodynamics parameters provided by the force field CHARMM are extremely high (-754 and -730 for tautomer one, compound 13 and tautomer two, compound 14 respectively).

**Phosphorylated compound 15 and 16 (regioisomer 1 and 2):** These compounds display as all phosphorylated compounds a high binding energy mainly due to electrostatic interaction. The total interaction energy values are close or higher to those observed that contain the imidazole ring. The total binding energy as calculated by CHARMM is slightly lower compared to the one observed for the phosphorylated imidazole containing compound and higher than the one of S1P (-697 and -702 vs -504).

**Phosphorylated compounds 17 and 18 (regioisomer 1 and 2):** These compounds display a high binding energy mainly due to electrostatic interaction. The value of the total interaction energy of compound 17 is close to those observed with the oxazole containing compounds. ΔG value is close as well to those observed with oxazole containing compounds but lower compared to those displaying by the imidazole ring. Interestingly, compound 18 displays a high interaction energy (-317), higher than the one observed for all investigated compounds. Compound 18 displays also a high ΔG binding energy as well as a high total binding energy as calculated by CHARMM (713).

### Conclusion:

Based on thermodynamics parameters it can be concluded:
1) Compounds 1 to 6: both oxazole and thiazole containing phosphorylated compounds have a higher S1PL inhibitory potency compared to the phosphorylated imidazole containing compounds.
2) Compounds 7 to 12: both oxazole and thiazole containing phosphorylated compounds have a higher S1PL inhibitory potency compared to the phosphorylated imidazole containing compounds.
3) Compounds 13 to 18: both oxazole and thiazole containing phosphorylated compounds have a similar S1PL inhibitory potency compared to the phosphorylated imidazole containing compounds. The thiazole derivative compound 18 displays the best binding parameters compared to the compounds 15, 16 and 17.

### Example 3

The following example is not according to the invention and is present for illustration purposes only.

Given the results described above, a more elaborate study was performed that compare more in depth the thermodynamic binding parameters of different S1P inhibitors (see Table 5 and Figure 3) against human and bacterial S1PL were further established. Two isomeric forms of each inhibitor were compared and for those with the carboxyl group of (ACB1907-10) additional evaluation of both the E and the Z-oxime was included in the analysis. This experimental approach assessed two mechanisms of inhibition: 1) S1P competition at the catalytic site or 2) pyridoxal-5-phosphate (PLP) cofactor displacement. An exhaustive MM-PBSA energy analysis of the library of compounds (Tables 5 and 6) used human S1PL (PDB code: 4Q6R), and Burkholderia pseudomallei-K96243 S1PL (PDB code:5K1R) crystal structures. Genetic Optimization for Ligand Docking (GOLD) algorithm and CHEMPLP7 (Empirical fitness functions optimized for pose prediction) scoring function that uses hydrogen bonding terms and multiple linear potentials to model Van der Waals and repulsive terms in conjunction with CHARMM and MM_PBSA energies. All of the compounds tested were assessed in terms of deltaG-PB which determines the affinity of the molecule binding into the active site while taking into account the effect of the solvent and the Total Interaction Energy which is the sum of the Van der Waals and electrostatic energies of each residue in the binding site with the bound ligand. In the initial study the solvation-desolvation effects were not considered as only one crystal structure and one binding site was analyzed. For this study additional analytical parameters incorporating the solvation-desolvation effect. This is encompassed within the MM-PBSA strategy where the binding energy is calculated by comparing the two states of the ligand, bound and unbound. Here, the ligand initially unbound and totally solvated by water, then to be partially desolvated before binding into the active site. This solvation-desolvation process, normalizes the binding energy when comparing different compounds, sites or receptors. This factor attributes to the difference in binding results obtained by the two studies.

Given the strong possibility that the compounds could be phosphorylated at the OH group, which is supported by observations of Ohtoyo et al, 2016, additional comparisons of binding affinities after phosphorylation was included.

All of the tested compounds within this series demonstrated the requirement of phosphorylation for SIPL-binding both in the PLP-cofactor domain and the S1P catalytic domain, indicating that they are pro-drugs and would require an additional metabolism to inhibit S1P and provide biological effects (Figure 1-1 A and 1-2 A, and Figure 3). Unexpectedly, for both binding sites, the inhibitors docked into the bacterial S1PL displayed significantly elevated total interaction energy over docking into the human S1PL whilst the DeltaG-PB remained relatively similar.

Classification of the binding energies according to their chemical family of imidazole compounds, thiazole compounds or oxazole compounds was performed for inhibitors bound into both the PLP and the S1P binding sites in both human and bacterial S1PL. Unexpectedly, a distinct cluster was identified that was classified as "low PLP binders" in the phosphorylated compounds bound to the PLP-cofactor binding site in the human S1PL with a total interaction energy less than-40 kcal/mol. Of this group, the most frequent were thiazole compounds (5/7). Identification of compounds that provide low PLP-cofactor binding improves the compound selectivity of the inhibitors for S1P over other PLP-cofactor dependent enzymes, minimizing undesired off-target effects.

Conversely, for compounds bound to the S1P catalytic domain, a cluster "High S1P binders" was identified with a total interaction energy > -75 kcal/mol in both S1PL of human and *B*. *pseudomallei.* Globally, within this group there were 6 thiazole compounds, 6 oxazole compounds and 5 imidazole compounds out of the 17 total, respectively. Yet, within the human S1PL, 4 / 6 compounds were thiazoles. Oxazole compounds were the most abundant chemical family for the modelling results with bacterial S1PL (5/11). Interestingly, the only phosphorylated imidazole compound found in the human S1PL modelling was phosphorylated form of ACB1913. And it was the only imidazole compound found in the "High S1P binders" clusters in both human and Burkholderia S1PL.

**Table 5: compounds used in modelling analysis**

| **Imidazole** | **Oxazole** | **Thiazole** |
|---|---|---|
| 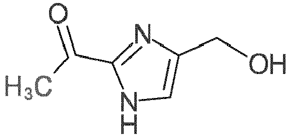 | 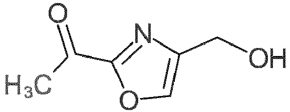 | 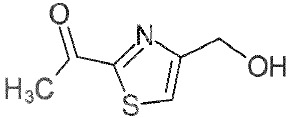 |
| ACB 1902 (A6770 tautomer1) | ACB1904 (regioisomer1) | ACB1906 (regioisomer1) |
| 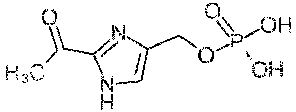 | 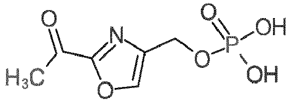 | 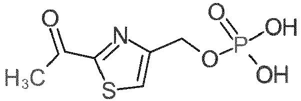 |
| ACB 1902-P (A6770-P tautomer1) | ACB1904-P (regioisomer1) | ACB1906-P (regioisomer1) |
| 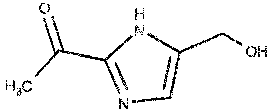 | 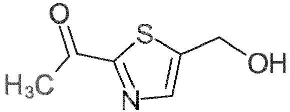 | 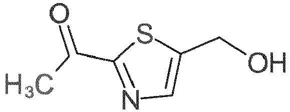 |
| ACB 1901 (A6770 tautomer2) | ACB1903 (regioisomer2) | ACB1905 (regioisomer2) |
| 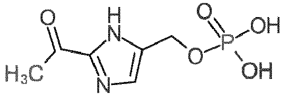 | 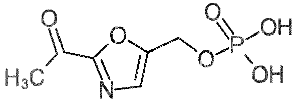 | 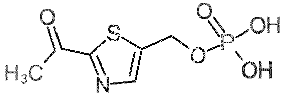 |
| ACB1901-P (tautomer2) | ACB1903-P (regioisomer2) | ACB1905-P (regioisomer2) |
| 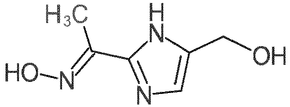 | 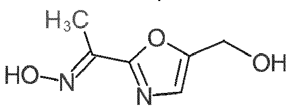 | 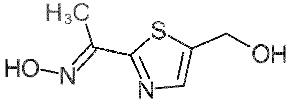 |
| ACB1907 (tautomer2_ e-oxime) | ACB1909 (regioisomer2_e-oxime) | ACB1911 (regioisomer2_e-oxime) |
| 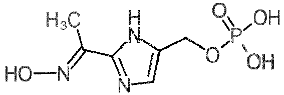 | 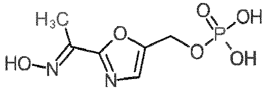 | 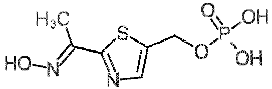 |
| ACB1907-P (tautomer2_ e-oxime) | ACB 1909-P (regioisomer2 e-oxime) | ACB1911-P (regioisomer2_e-oxime) |
| 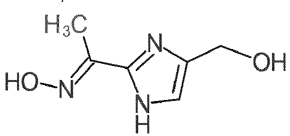 | 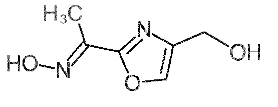 | 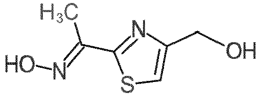 |
| ACB1908 (tautomer1_e-oxime) | ACB1910 (regioisomer1**_**e-oxime) | ACB1912 (regioisomer1_e-oxime) |
| 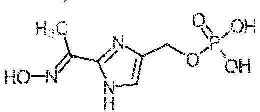 | 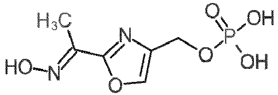 | 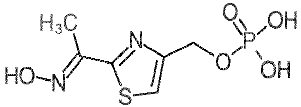 |
| ACB1908-P (tautomer 1_ e-oxime) | ACB1910-P (regioisomer1 e-oxime) | ACB1912-P (regioisomer1_e-oxime) |
| | | |

| **Imidazole** | **Oxazole** | **Thiazole** |
|---|---|---|
| 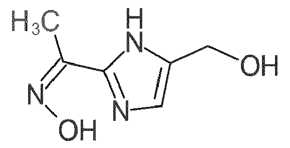 | 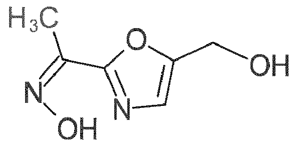 | 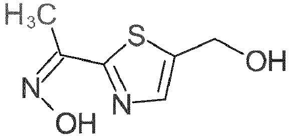 |
| ACB1907z (tautomer2z-oxime) | ACB1909z (regioisomer2z-oxime) | ACB1911z (regioisomer2z-oxime) |
| 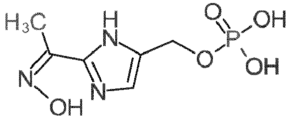 | 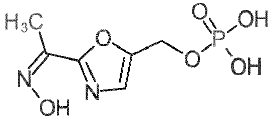 | 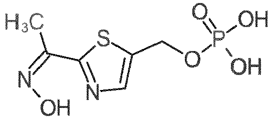 |
| ACB1907-Pz (tautomer2z-oxime) | ACB 1909-P (regioisomer2z-oxime) | ACB1911-Pz (regioisomer2_z-oxime) |
| 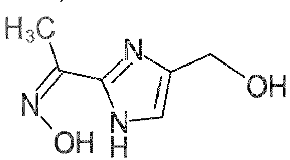 | 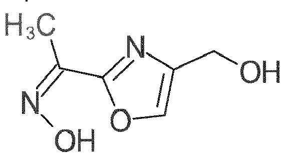 | 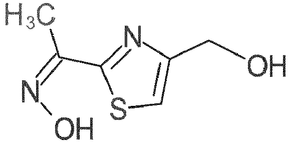 |
| ACB1908z(tautomer1z-oxime) | ACB1910z (regioisomer1z-oxime) | ACB1912z (regioisomer1_z-oxime) |
| 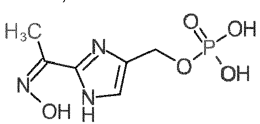 | 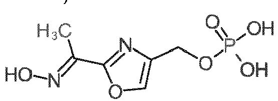 | 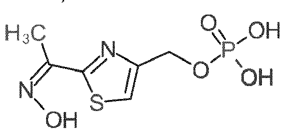 |
| ACB1908-Pz(tautomer1z-oxime) | ACB1910-Pz (regioisomerlz-oxime) | ACB1912-Pz (regioisomer1_z-oxime) |
| 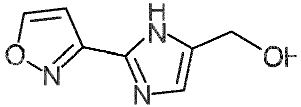 | 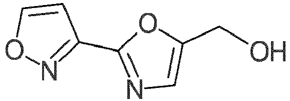 | 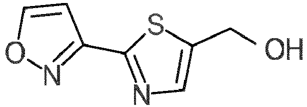 |
| ACB1919 (tautomer2) | ACB1921 (regioisomer2) | ACB1923 (regioisomer2) |
| 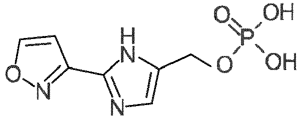 | 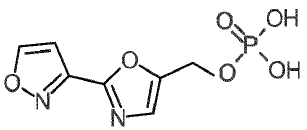 | 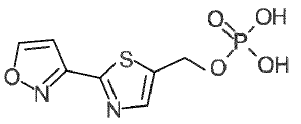 |
| ACB1919-P (tautomer2) | ACB1921-P (regioisomer2) | ACB1923-P (regioisomer2) |
| 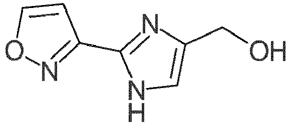 | 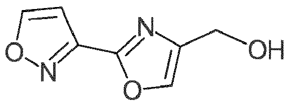 | 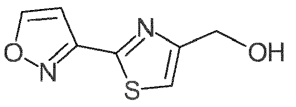 |
| ACB1920 (tautomer 1) | ACB1922 (regioisomer1) | ACB1924 (regioisomer 1) |
| 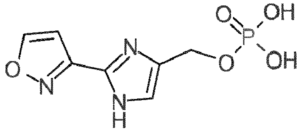 | 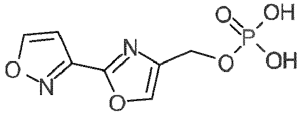 | 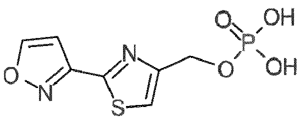 |
| ACB1920-P (tautomer1) | ACB1922-P (regioisomer1) | ACB1924-P (regioisomer1) |
| 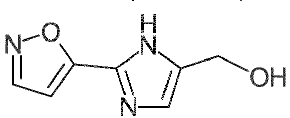 | 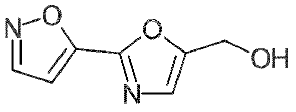 | 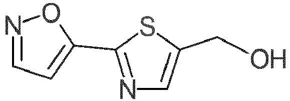 |
| ACB1913 (tautomer 1) | ACB1915 (regioisomer1) | ACB1917 (regioisomer1) |
| 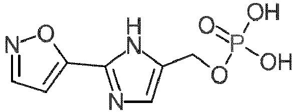 | 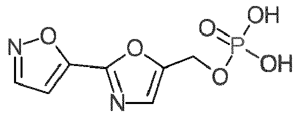 | 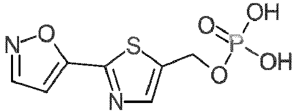 |
| ACB1913-P (tautomer 1) | ACB1915-P (regioisomer1) | ACB1917 (regioisomer1) |
| 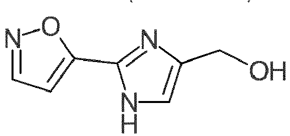 | 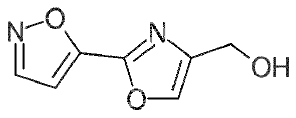 | 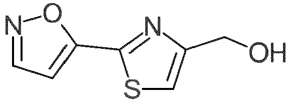 |
| ACB1914 (tautomer 2) | ACB1916 (regioisomer2) | ACB1918 (regioisomer2) |
| 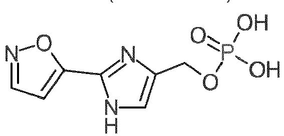 | 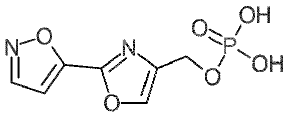 | 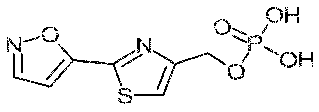 |
| ACB1914-P (tautomer 2) | ACB1916-P (regioisomer2) | ACB1918-P (regioisomer2) |
| 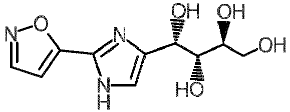 | 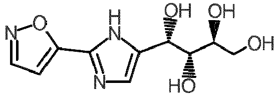 | 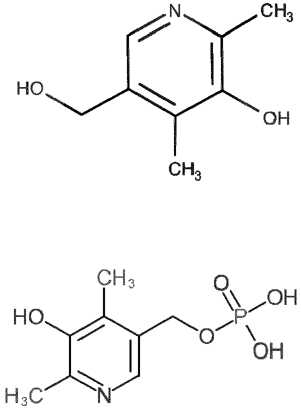 |
| ACB2008 (tautomer 1) | ACB2008 (tautomer 2) | 4-Deoxypyridoxine (4DOP) /4-DOP-P |

### Conclusion:

The modelling data with the *B. pseudomallei* S1PL demonstrate that the compounds of the invention are good S1P inhibition candidates. This clearly demonstrates that these compounds may be used to treat infectious disease, not only through the modulation of host S1P-dependent responses (including those of the immune response), but can target S1P responses in selected pathogens and act as an antibiotic. Moreover, unlike in the case for the inhibition targeting human S1PL, the observed higher binding affinity of the molecules B.p in the PLP-binding pocket in conjunction with their ability to interact with the S1P catalytic sites confers an additional advantage for the application in infectious diseases.

### Example 4

The following example is not according to the invention and is present for illustration purposes only.

Given that the modelling data demonstrated increased binding affinity of the phosphorylated compounds over the pro-drugs, ACB1906 was synthetized. The phosphorylated form of this compound was found in the "high S1P binders" cluster in the modelling analysis. ACB1906's ability to be phosphorylated by recombinant human Pyridoxal kinase (PDXK) *in vitro* was compared to the structurally similar known imidazole S1PL inhibitor A6770, the PDXK and S1PL non-specific inhibitor 4-deoxypyridoxine (4-DOP) and the natural pyridoxal ligand. PDXK was already reported to phosphorylate the THI-derived imidazole compounds A6770 to A6770-P. Briefly, the compounds were incubated with the rhPDXK in the presence of ATP and after 1 hour incubation the quantification of ADP formation was measured by a luminescence-based assay. As can be seen by Figure 2, the novel compound ACB1906 displays a significantly increased ability to be phosphorylated in comparison to A6770 (~2-fold). Increased phosphorylation of thiazole compounds confers an advantage in therapeutic applications as this would be a limiting step for its ability to effect a therapeutically relevant modulation in S1P levels.

## Claims

1. A compound 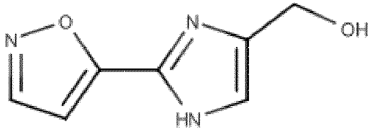 or phosphorylated derivatives thereof and/or phosphoric acid esters thereof.

2. A pharmaceutical composition comprising the compound of claim 1 and pharmaceutically acceptable excipients and/or carriers.

3. The compound of claim 1 for use in the treatment, prevention or suppression of diseases and disorders known to be susceptible to variations in the levels of sphingosine-1-phosphate, 2-hexadecanal levels and phosphoethanolaminea.

4. The compound of claim 1 for use in a method of preventing and/or treating infectious diseases in a subject, wherein the infectious diseases are caused by pathogenic microorganisms selected from the group comprising bacteria, viruses, fungi and parasites.

5. The compound for use according to claim 4, wherein the infectious diseases are selected from the group comprising pulmonary infections, plague, systemic infections and parasitic diseases.

6. The compound for use according to claim 5, wherein the pulmonary infections are selected from the group comprising virus-induced infections, such as Influenza, Respiratory syncytial virus, SARS-CoV, Bacteria-induced pneumonia and Tuberculosis.

7. The compound for use according to claim 5, wherein the systemic infections are selected from the group comprising Sepsis and those induced by Cytomegalovirus, Meliodosis, Legionnaire's disease, Dengue, Chikagunya, Measles, Candidiasis.

8. The compound for use according to claim 5, wherein the parasitic diseases are selected from the group comprising Leishmaniasis, Human African Trypanosomiasis, Chagas disease Schistosomiasis, Echinococcosis, and Malaria.

9. The compound for use according to any one of claims 4-8, wherein the method further comprises administering to the subject one or more additional active agents selected from the group comprising antibiotics, anti-bacterial agents, antifungal agents, antiparasitic agents, osmotic diuretics, anti-convulsants, anti-pyretics, immunomodulating drugs, anti-viral agents and combinations thereof.

10. The compound for use according to any one of claims 3-9, wherein the subject is selected from the group consisting of a mammal, a fish, a bird, and a crustacean.

11. The compound of claim 1 for use in a method of treatment of cancer, wherein a therapeutically effective amount of the compound of claim 1 is administered to a subject prior to or during the chemotherapy and/or the radiotherapy.

## Patentansprüche

1. Verbindung 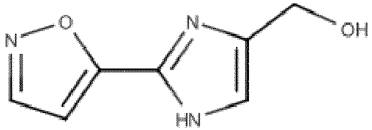 oder phosphorylierte Derivate davon und/oder Phosphorsäureester davon.

2. Pharmazeutische Zusammensetzung, umfassend die Verbindung nach Anspruch 1 und pharmazeutisch annehmbare Hilfsstoffe und/oder Träger.

3. Verbindung nach Anspruch 1 zur Verwendung bei der Behandlung, Vorbeugung oder Unterdrückung von Krankheiten und Störungen, von denen bekannt ist, dass sie für Schwankungen der Sphingosin-1-phosphat-, 2-Hexadecanal- und Phosphoethanolamin-Spiegel anfällig sind.

4. Verbindung nach Anspruch 1 zur Verwendung in einem Verfahren zur Vorbeugung und/oder Behandlung von Infektionskrankheiten bei einem Subjekt, wobei die Infektionskrankheiten durch pathogene Mikroorganismen verursacht sind, die aus der Gruppe ausgewählt sind, die Bakterien, Viren, Pilze und Parasiten umfasst.

5. Verbindung zur Verwendung nach Anspruch 4, wobei die Infektionskrankheiten aus der Gruppe ausgewählt sind, die Lungeninfektionen, Pest, systemische Infektionen und Parasiten umfasst.

6. Verbindung zur Verwendung nach Anspruch 5, wobei die Lungeninfektionen aus der Gruppe ausgewählt sind, die virusinduzierte Infektionen, wie Influenza, Respiratorisches Synzytialvirus, SARS-CoV, bakteriell induzierte Lungenentzündung und Tuberkulose umfasst.

7. Verbindung zur Verwendung nach Anspruch 5, wobei die systemischen Infektionen aus der Gruppe ausgewählt sind, die Sepsis und solche, die durch Cytomegalovirus induziert werden, Meliodose, Legionärskrankheit, Dengue, Chikagunya, Masern und Candidiasis umfasst.

8. Verbindung zur Verwendung nach Anspruch 5, wobei die parasitären Krankheiten aus der Gruppe ausgewählt sind, die Leishmaniose, menschliche afrikanische Trypanosomiasis, Chagas-Krankheit, Schistosomiasis, Echinokokkose und Malaria umfasst.

9. Verbindung zur Verwendung nach einem der Ansprüche 4 bis 8, wobei das Verfahren ferner die Verabreichung eines oder mehrerer zusätzlicher Wirkstoffe an das Subjekt umfasst, die aus der Gruppe ausgewählt sind, die Antibiotika, antibakterielle Mittel, antimykotische Mittel, antiparasitäre Mittel, osmotische Diuretika, krampflösende Mittel, fiebersenkende Mittel, immunmodulierende Medikamente, antivirale Mittel und Kombinationen davon umfasst.

10. Verbindung zur Verwendung nach einem der Ansprüche 3 bis 9, wobei das Subjekt aus der Gruppe ausgewählt ist, die aus einem Säugetier, einem Fisch, einem Vogel und einem Krebstier besteht.

11. Verbindung nach Anspruch 1 zur Verwendung in einem Verfahren zur Behandlung von Krebs, wobei eine therapeutisch wirksame Menge der Verbindung nach Anspruch 1 einem Subjekt vor oder während der Chemotherapie und/oder der Strahlentherapie verabreicht wird.

## Revendications

1. Composé 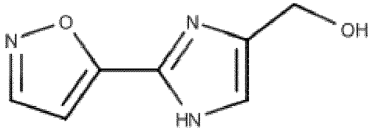 ou ses dérivés phosphorylés et/ou ses esters d'acide phosphorique.

2. Composition pharmaceutique comprenant le composé de la revendication 1 et des excipients et/ou porteurs pharmaceutiquement acceptables.

3. Composé de la revendication 1 pour utilisation dans le traitement, la prévention ou la suppression de maladies et de troubles connus pour être sensibles aux variations des taux de sphingosine-1-phosphate, de 2-hexadécanal et de phosphoéthanolamine.

4. Composé de la revendication 1 pour utilisation dans une méthode de prévention et/ou de traitement de maladies infectieuses chez un sujet, dans lequel les maladies infectieuses sont provoquées par des micro-organismes pathogènes choisis entre le groupe comprenant des bactéries, des virus, des champignons et des parasites.

5. Composé pour utilisation selon la revendication 4, dans lequel les maladies infectieuses sont choisies entre le groupe comprenant les infections pulmonaires, la peste, les infections systémiques et les maladies parasitaires.

6. Composé pour utilisation selon la revendication 5, dans lequel les infections pulmonaires sont choisies entre le groupe comprenant les infections induites par des virus, telles que la grippe, le virus respiratoire syncytial, le SRAS-CoV, la pneumonie induite par des bactéries et la tuberculose.

7. Composé pour utilisation selon la revendication 5, dans lequel les infections systémiques sont choisies entre le groupe comprenant le sepsis et celles induites par le cytomégalovirus, la mélioïdose, la maladie du Légionnaire, la dengue, le chikungunya, la rougeole, la candidose.

8. Composé pour utilisation selon la revendication 5, dans lequel les maladies parasitaires sont choisies entre le groupe comprenant la leishmaniose, la trypanosomiase humaine africaine, la maladie de Chagas, la schistosomiase, l'échinococcose et le paludisme.

9. Composé pour utilisation selon l'une quelconque des revendications 4 à 8, dans lequel le procédé comprend en outre l'administration au sujet d'un ou de plusieurs agents actifs supplémentaires choisis entre le groupe comprenant des antibiotiques, des agents antibactériens, des agents antifongiques, des agents antiparasitaires, des diurétiques osmotiques, des anticonvulsivants, des antipyrétiques, des médicaments immunomodulateurs, des agents antiviraux et leurs combinaisons.

10. Composé pour utilisation selon l'une quelconque des revendications 3 à 9, dans lequel le sujet est choisi entre le groupe constitué d'un mammifère, d'un poisson, d'un oiseau et d'un crustacé.

11. Composé de la revendication 1 pour utilisation dans une méthode de traitement du cancer, dans lequel une quantité thérapeutiquement efficace du composé de la revendication 1 est administrée à un sujet avant ou pendant la chimiothérapie et/ou la radiothérapie.
